# EUROPEAN PATENT SPECIFICATION

(11) **EP 1 003 575 B1**
(45) Date of publication and mention of the grant of the patent: **27.10.2004**
(21) Application number: 98943208.3
(22) Date of filing: 14.08.1998
(51) Int. Cl.: A61M 1/36

(54) **SYSTEM FOR MINIMALLY INVASIVE SURGERY WITH VACUUM-ASSISTED VENOUS DRAINAGE**
SYSTEM FÜR DIE MINIMAL INVASIVE CHIRURGIE MIT VAKUUMUNTERSTÜTZTER VENÖSER DRAINAGE
SYSTEME D'OPERATION LA MOINS EFFRACTIVE POSSIBLE A DRAINAGE VEINEUX A DEPRESSION

(30) Priority: 15.08.1997 US 911870; 26.09.1997 US 938058
(43) Date of publication of application: 31.05.2000
(73) Proprietor: BAXTER INTERNATIONAL INC., Deerfield, Illinois 60015 (US); THE CLEVELAND CLINIC FOUNDATION, Cleveland, OH 44195 (US)
(72) Inventor: COSGROVE, Delos, M., Hunting Valley, OH 44022 (US); FOSTER, Robert, C., Rocky River, OH 44106 (US); CAMBRON, Ronald, Laguna Hills, CA 92653 (US); VIJAY, Francis, Lake Forest, CA 92630 (US); KNIGHT, Richard, Huntington Beach, CA 92646 (US); LITZIE, Ken, Irvine, CA 92620 (US)
(74) Representative: Baker, Colin John
(86) International application number: PCT/US1998/016893
(87) International publication number: WO 1999/008734

(56) References cited:
- EP-A- 0 089 748
- WO-A-96/24397

## Description

### Field of the Invention

The present invention relates generally to a reduced prime volume cardiopulmonary bypass system that is useful in connection with minimally invasive cardiovascular surgical procedures, and more specifically, vacuum-assisted venous drainage systems and associated minimally invasive surgical methods.

### Background of the Invention

Various cardiovascular surgical procedures, including repair or replacement of aortic, mitral and other heart valves, repair of congenital defects, coronary artery bypass grafting, and treatment of aneurysms, involve arrest of cardiac function. In such procedures, a cardiopulmonary bypass ("CPB") system must be used to oxygenate the blood and maintain circulation of the oxygenated blood through the patient during the entire time the heart is arrested. Typically, the components of the CPB system include, in sequence, one or more cannulae which are inserted into one or more major veins, such as the inferior vena cava, or into the heart itself for draining or withdrawing deoxygenated blood from the patient, a venous reservoir for collecting the venous blood, an oxygenator for removing CO₂ from and oxygenating the deoxygenated blood, a filtration unit, an arterial pump for pumping the oxygenated blood back into the patient, and a cannulae which is inserted into a major artery such as the aorta or femoral artery for delivering oxygenated blood to the patient. The components of the CPB system additionally include tubing for carrying the blood throughout the CPB system. Commonly, a sucker extracts excess fluid from the chest cavity during the operation and diverts the fluid, which may contain bone chips or other particulates, into the top of a cardiotomy reservoir. The cardiotomy sucker pulls pooled blood from the chest cavity using a vacuum which may be generated by a roller pump, for example. In addition, a vent cannula may be positioned in the heart for suctioning other fluids during the operation, those fluids also being directed to the cardiotomy reservoir through a roller pump. The fluid entering the cardiotomy reservoir is first filtered before being combined with the venous blood in the venous reservoir. Often, the cardiotomy reservoir is integrally formed with the venous reservoir.

In contrast to the cardiotomy and vent lines, the venous blood collection cannula is positioned in a vein in contact with a relatively constant stream of blood. Thus, the conventional venous drain method is to place the reservoir under the patient and siphon blood by gravity. This method is facilitated by the relatively large bore venous return cannulae of 36 French OD or more used in open-heart surgery. A major drawback to the gravity drain, however, is that the system must be primed before a return pump downstream of the reservoir can take effect. The only means of enhancing venous return is by increasing the head height between the cannula and the venous reservoir. This is achieved either by lowering the location of the reservoir, limited by the floor, and/or by raising the level of the operating table, limited by the practical needs of the operating room. Moreover, a considerable length of tubing, typically about 1-02m to 2-03m (40 to 80 inches), is needed to connect the patient to the reservoir and to the arterial pump. Thus, the patient's blood interacts with a large surface area of tubing which increases the possibility of hemodilution, where the tubing is primed, as well as hemolysis. Large venous blood collection cannulae also may cause difficulties for surgeons using recently developed minimally invasive procedures for cardiovascular surgery. In such procedures, a small incision, typically 10 cm, is made to gain access to the heart. If venous cannulae having a large diameter are inserted through this incision, the remaining working space available to the surgeon is significantly reduced.

In conventional perfusion systems, the conduits leading from the patient to the various components of the system contain a significant volume of blood. In addition, the various components such as the venous cardiotomy reservoir and arterial filter also require a certain volume of blood to function properly. All of these components put together require a certain "prime" or volume of blood from the patient to function. The prime volume can be defined as that volume of blood outside the patient, or extracorporeal. Often, over 40% of the priming volume, or as much as 2400 ml, may be used to fill tubing. High priming volumes may dilute the patient's red blood cells, platelets, and plasma proteins. Such hemodilution may lead to complications during recovery. For example, patients having diluted platelets and plasma proteins may be more likely to suffer from bleeding problems due to inadequate coagulation. Such patients may be more likely to suffer from post-operative anemia and thus require transfusions.

One type of perfusion system uses a vacuum in conjunction with gravity to drain blood from the venous system. Such a configuration is the subject of a paper entitled "Trial of Roller Pump-Less Cardiopulmonary Bypass System" by Hiroura, et al. of the Department of Thoracic Surgery, Nagoya University School of Medicine, published in conjunction with Owari Prefectural Hospital, both in Aichi. Japan. This reference discloses a system in which a wall vacuum generates a negative pressure of between - 667 and -4666 Pa (-5 and -35 mmHg) within a main reservoir, which is connected to a plurality of individual suction reservoirs and to a venous return line. Cardiotomy and other suction lines from the patient are attached to the individual suction reservoirs, and the vacuum pressure within each one of the suction reservoirs can be regulated independently. The system further includes a centrifugal pump under the main reservoir for pumping blood through the rest of the CPB system and to the patient. A significant amount of hardware is needed for this system to regulate and connect the various pressure chambers, and there is a large blood/surface contact area in comparison to other systems. The Hirowa device is described in EP-A-786,261.

Another use of vacuum in venous drainage is seen in WO-A-96/24397. This publication discloses a hard-shelled venous reservoir within which a vacuum is developed using centrifugal pump attached to the bottom of the reservoir. The vacuum in the reservoir may be supplemented with a secondary wall vacuum.

Another technique for augmenting venous drainage uses a centrifugal pump connected to the venous line as described in L. Solomon et al., *Augmented Femoral Venous Return*, Ann. Thorac. Surg. (1993) 55:1262-3. Typically, in this technique, a venous cannulae is inserted into the right femoral vein and then guided to and positioned in the right atrium. The arterial portion of the vascular system is accessed through a cannulae which is inserted into the left femoral artery. Unfortunately, such femoral-femoral cannulation can lead to complications such as thrombophlebitis, wound infections, and dissections.

Various types of surgical procedures are performed on the heart and the great vessels. Many of such procedures, particularly those involving the aorta, and aortic valve employ a gross thoracotomy, e.g., a median sternotomy, in order to gain access to the involved portion of the heart or vessel. In other words, the procedures entail traumatically splitting open the patient's chest. Incisional pain tends to require significant postoperative analgesia and postoperative discomfort tends to result in significant patient morbidity and lengthy hospital stays. In addition, because the pericardial sac is opened underlying the sternum, after the procedure the heart has a tendency to become adherent to the sternum. This can be problematic in the event of subsequent procedures.

The desirability of avoiding the use of median stemotomy, and other gross thoracotomy procedures, in connection with surgery on the heart and the great vessels has been recognized. For example, techniques have been proposed in which a scope is inserted through a percutaneous intercostal penetration in the patient's chest (an incision between the ribs) to observe internal procedures performed by instruments introduced into the chest with the scope, or through cannulae disposed in other intercostal spaces, i.e., between two adjacent ribs. Such techniques and instruments for performing such techniques within the heart and great vessels is described in International Publication WO 95/15715 by Sterman, et al., published June 15, 1995. However, such techniques require special instrumentation and special skills to perform, and may extend the time the heart is arrested and the duration of the procedure.

### Summary of the Invention

The present invention provides a cardiopulmonary by-pass system comprising a first cannula for providing arterial return blood supply to the patient, a second cannula for providing venous drainage from the patient, a venous reservoir closed to atmosphere having a blood inlet connected to said second cannula, and a vacuum supply connected to said venous reservoir for providing a predetermined desired vacuum pressure range within said venous reservoir, said second cannula having a reduced cross-sectional area so as to minimize the space consumed by said second cannula when placed within a surgical field, said reduced cross-sectional area sized to maintain sufficient drainage from said patient under said vacuum pressure range within said venous reservoir.

The venous reservoir may be hard-shelled with a blood outlet for removing blood from the reservoir and a vacuum inlet for supplying a vacuum to the reservoir. A vacuum regulator subassembly for manually setting said predetermined desired vacuum pressure range is desirable. The predetermined desired vacuum supplied to said venous reservoir is preferably approximately -9332 Pa to -3333 Pa (-70 to -25 mmHg). A valve subassembly may be used to manually enable and disable a vacuum from being supplied to said reservoir.

The venous reservoir may comprise a flexible, blood impermeable container within a rigid, sealed outer housing, with a conduit attached to the blood inlet port and in communication with the interior of the reservoir, the conduit passing through a sealed opening in the housing and being connected to a source of venous blood. In this embodiment, a second conduit preferably extends between the vacuum source and the interior of the housing through a sealed opening, and a pressure regulator is provided between the vacuum conduit and vacuum source. Preferably, an air permeable membrane forms a portion of the flexible container for venting air from within the container to the housing interior.

Further objects and advantages of the present invention shall become apparent to those skilled in the art upon reading and understanding the following detailed description of a presently preferred embodiment of the invention.

### Brief Description of the Drawings.

Figure 1 schematically illustrates a cardiopulmonary bypass (CPB) circuit with the vacuum-assisted venous drainage system of the present application.
Figure 2 schematically illustrates portions of the vacuum line, reservoir, oxygenation unit, and filtration unit of Figure 1.
Figure 3 schematically illustrates the vacuum regulator and valve subassemblies of Figure 1.
Figure 4 schematically illustrates the valve subassemblies and roller pumps of Figure 1.
Figure 5 schematically illustrates an alternate embodiment of the vacuum-assisted venous drainage system in a CPB circuit.
Figure 6 schematically illustrates the vent and suction reservoir subassemblies of the embodiment of Figure 5.
Figure 7a is a cross-sectional view of a hard-shelled venous reservoir adapted for vacuum-assisted venous drainage and having reduced blood/air interface, prior to a vacuum being applied;
Figure 7b is a cross-sectional view of the reservoir of Figure 7a after a vacuum is applied;
Figure 8a is a cross-sectional view of another embodiment of a hard-shelled venous reservoir adapted for vacuum-assisted venous drainage and having reduced blood/air interface, prior to a vacuum being applied;
Figure 8b is a cross-sectional view of the reservoir of Figure 8a after a vacuum is applied;
Figure 9 schematically illustrates a further embodiment of a minimally invasive CPB circuit of the present invention utilizing a vacuum-assisted soft-shell venous reservoir system;
Figure 10 schematically illustrates a combination pressure-relief valve and vacuum stabilizing device for use with vacuum-assisted reservoirs of the present invention;
Figures 11 and 11A schematically illustrate a human chest and the disposition of a right parastemal incision in connection with an aortic surgery procedure in accordance with the present invention;
Figure 12 pictorially illustrates the right parasternal incision of Figure 11 showing respective costal cartilages;
Figure 13 pictorially illustrates the right parasternal incision of Figure 11 after respective costal cartilage units are excised and the incision retracted;
Figure 14 schematically illustrates the disposition of respective by-pass cannula employed in connection with an aortic surgery procedure in accordance with the present invention;
Figure 15 schematically illustrates an alternative disposition of respective by-pass cannula employed in connection with an aortic surgery procedure in accordance with the present invention;
Figure 16 pictorially illustrates the right parastemal incision of Figure 11 after the aorta is opened to expose the aortic valve;
Figure 17 pictorially illustrates the injection of cardioplegia into the coronary ostia;
Figure 18 pictorially illustrates the right parasternal incision of Figure 11 after the aortic valve is removed, with traction sutures placed at the commissures;
Figure 19 pictorially illustrates insertion of an aortic valve prosthesis;
Figure 20 pictorially illustrates closure of the aorta;
Figure 21 pictorially illustrates disposition of temporary pacer leads and drainage tube;
Figure 22 pictorially illustrates a right parastemal incision after respective costal cartilage units are excised and the incision retracted;
Figure 23 pictorially illustrates the surgery field of Figure 22 after an incision of the right atrium;
Figure 23A pictorially illustrates an alternative way of occluding the aorta;
Figure 23B pictorially illustrates a second alternative way of occluding the aorta;
Figure 24 pictorially illustrates the surgical field of Figure 22 after an incision of the inter-atrial wall;
Figure 25 pictorially illustrates the surgical field of Figure 22 after the tissue has been retracted.
Figure 25A pictorially illustrates an alternative method of exposing the surgical field of Figure 25;
Figure 25B is a plan view of a flexible ring as seen in use in Figure 25A;
Figure 26 pictorially illustrates one step in an annuloplasty procedure in the surgical field of Figure 25;
Figure 27 pictorially illustrates another step in the annuloplasty procedure of Figure 26;
Figure 28 pictorially illustrates completion of the annuloplasty procedure of Figure 26;
Figure 29 pictorially illustrates the closure of the inter-atrial wall as incised in Figure 24;
Figure 30 pictorially illustrates the closure of the right atrium as shown incised in Figure 25;
Figure 31 pictorially illustrates a transverse incision across the sternum;
Figure 32 pictorially illustrates the exposed surgical field after the incision of Figure 31;
Figure 33 pictorially illustrates an incised aorta in the surgical field of Figure 32;
Figure 34 pictorially illustrates a surgical procedure on the aortic valve in the surgical field of Figure 32;
Figure 35 pictorially illustrates the replacement of an aortic valve in the surgical field of Figure 32;
Figure 36 pictorially illustrates the closure of the aorta in the surgical field of Figure 32; and;
Figure 37 pictorially illustrates the surgical field of Figure 32 after completion of the surgery;
Figure 38 schematically illustrate a human chest and another preferred embodiment of an incision in connection with heart surgery in accordance with the present invention;
Figure 39 pictorially illustrates the surgical field of Figure 38 after the tissue has been retracted and at the point of flooding the field with carbon dioxide;
Figure 40 pictorially illustrates an incised aorta in the surgical field of Figure 32;
Figure 41 pictorially illustrates the injection of cardioplegia into the coronary ostia;
Figure 42 pictorially illustrates the insertion of an aortic valve prosthesis;
Figure 43 pictorially illustrates closure of the aorta;
Figure 44 pictorially illustrates disposition of temporary pacer leads;
Figure 45 pictorially illustrates disposition of drainage tubes;
Figure 46 pictorially illustrates the surgical field of Figure 38 prior to incision of the right atrium;
Figure 47 pictorially illustrates the surgical field of Figure 38 prior to the incision of the inter-atrial wall;
Figure 48 pictorially illustrates the surgical field of Figure 47 after the tissue has been retracted;
Figure 49 is a cross-sectional view of the left ventricle after the interatrial wall has been incised and partially retracted;
Figure 50 is a cross-sectional view of the left ventricle after it has been fully retracted;
Figure 51 pictorially illustrates the surgical field of Figure 38 with exposure to the mitral valve;
Figure 52 illustrates a step in an annuloplasty procedure for the surgical field of Figure 38;
Figure 53 illustrates completion of the annuloplasty procedure of Figure 52;
Figure 54 illustrates closure of the inter-atrial wall as incised in Figure 47; and,
Figure 55 illustrates disposition of temporary pacer leads.

### Description of the Preferred Embodiments

The present invention provides vacuum-assisted venous blood drainage into reservoirs of various types. As shown and described herein, both hard- and soft-shelled reservoirs may be used, although those of skill in the art will recognize that various other types of reservoirs may also be adapted for vacuum-assisted drainage. Furthermore, the reservoirs as depicted in the present invention are combined cardiotomy and venous reservoirs, but venous reservoirs separate from cardiotomy reservoirs may also be adapted for vacuum-assisted drainage. Finally, various components of conventional cardiopulmonary bypass (CPB) systems may be used with the vacuum-assisted venous drainage reservoirs of the present invention, so that those illustrated are not to be considered limiting, and any other conventional components typically used in CPB systems and omitted from the description and drawings may be included.

In minimally invasive surgery, the chest cavity is not opened, but instead the various instruments and fluid communication conduits are inserted into the chest cavity through one or more small openings therein. Minimally invasive surgery greatly reduces the recovery time for such heart surgeries, and is rapidly gaining popularity in the medical community, and in the population at large. Although the present invention is illustrated in conjunction with minimally invasive surgery, similar advantages of reduced prime volume and less blood trauma are realized when using the invention in conjunction with more conventional open heart surgery techniques.

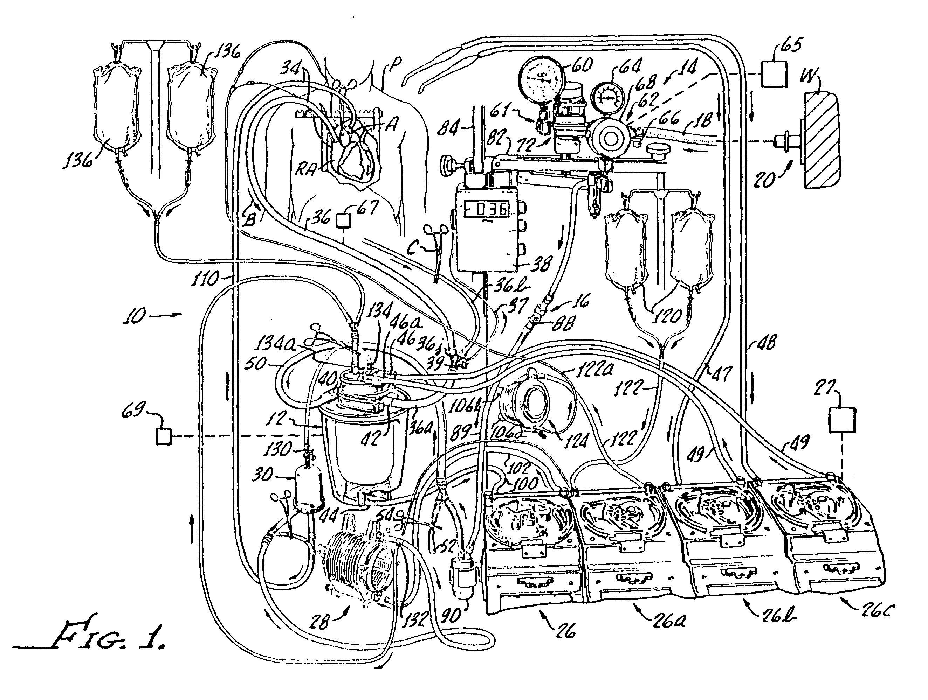

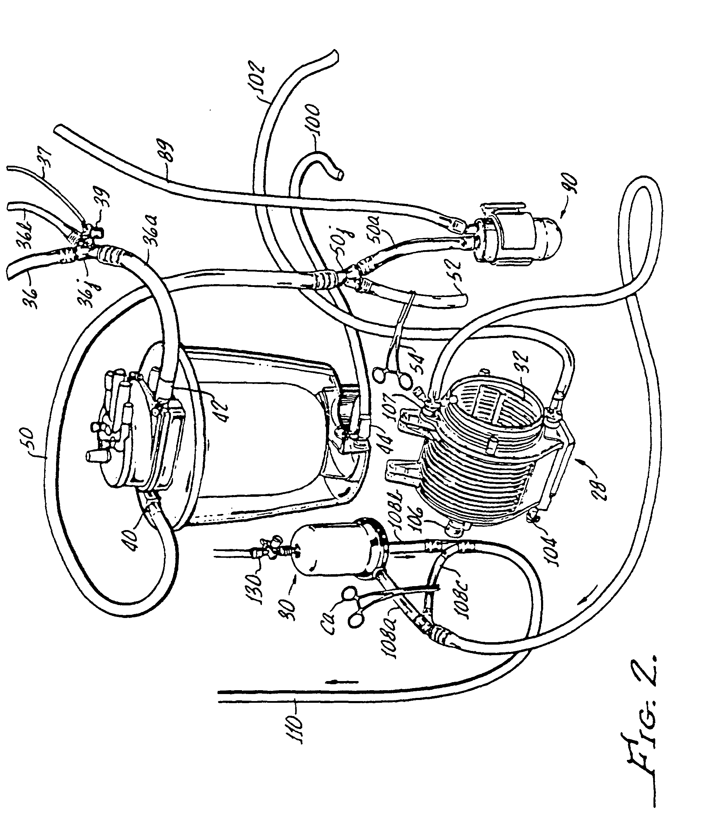

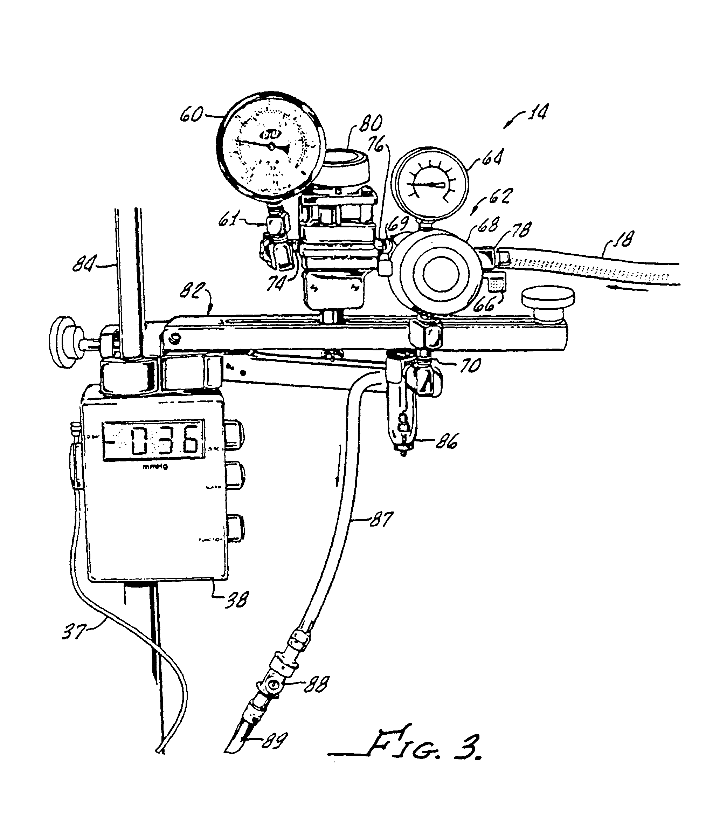

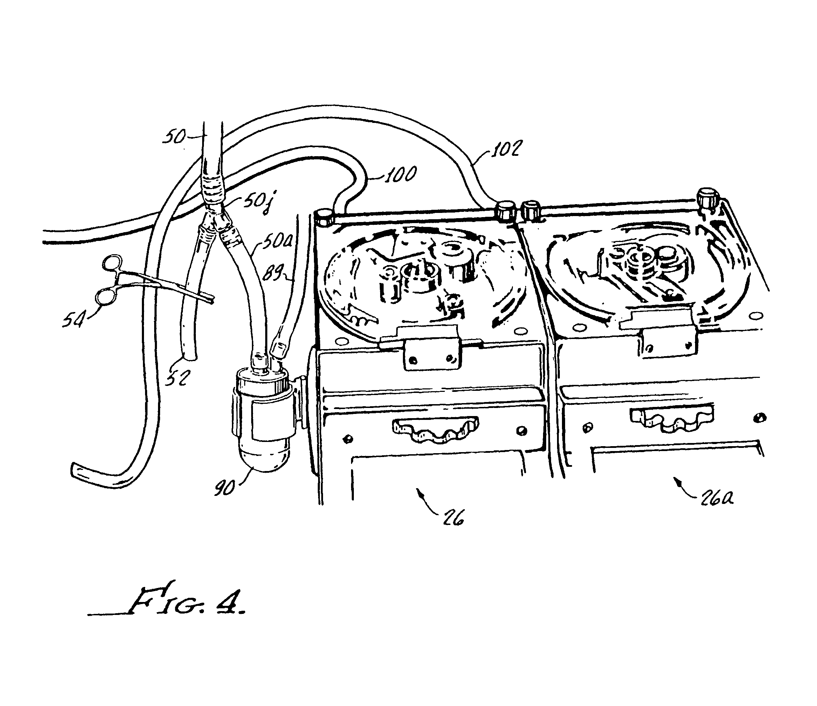

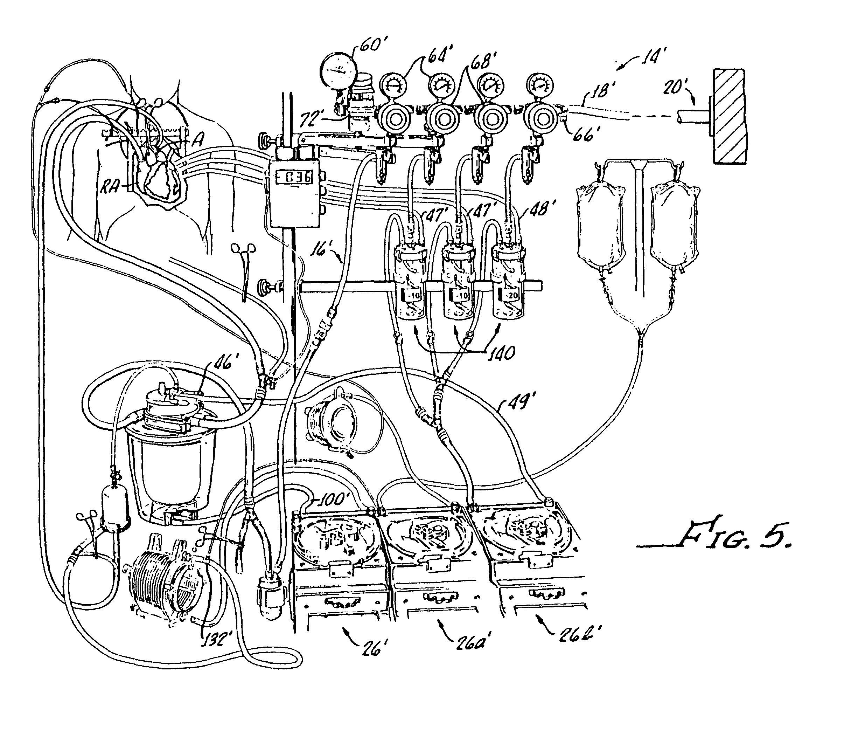

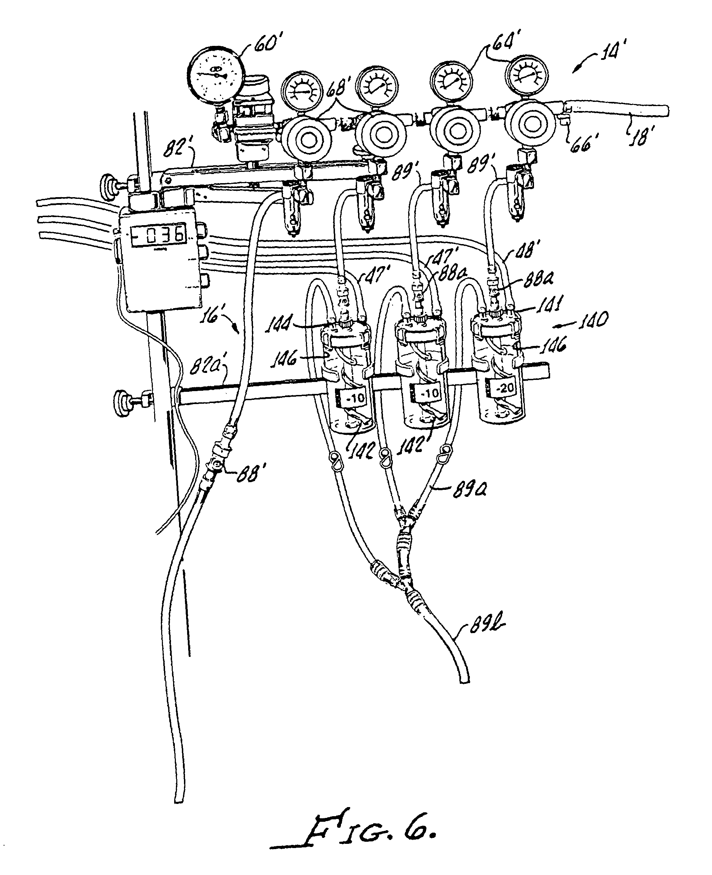

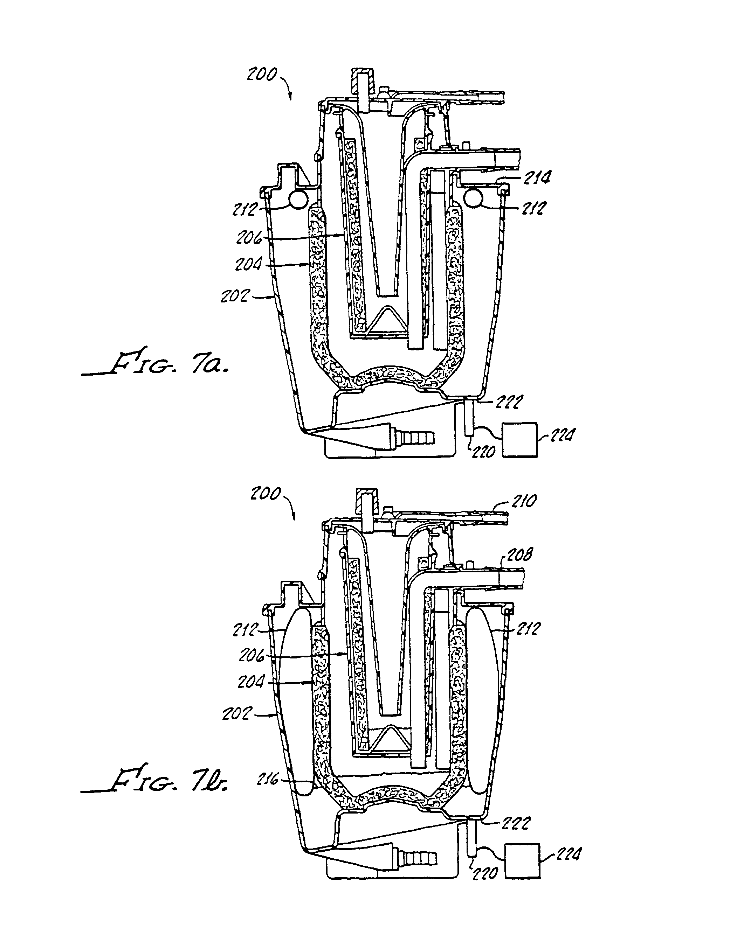

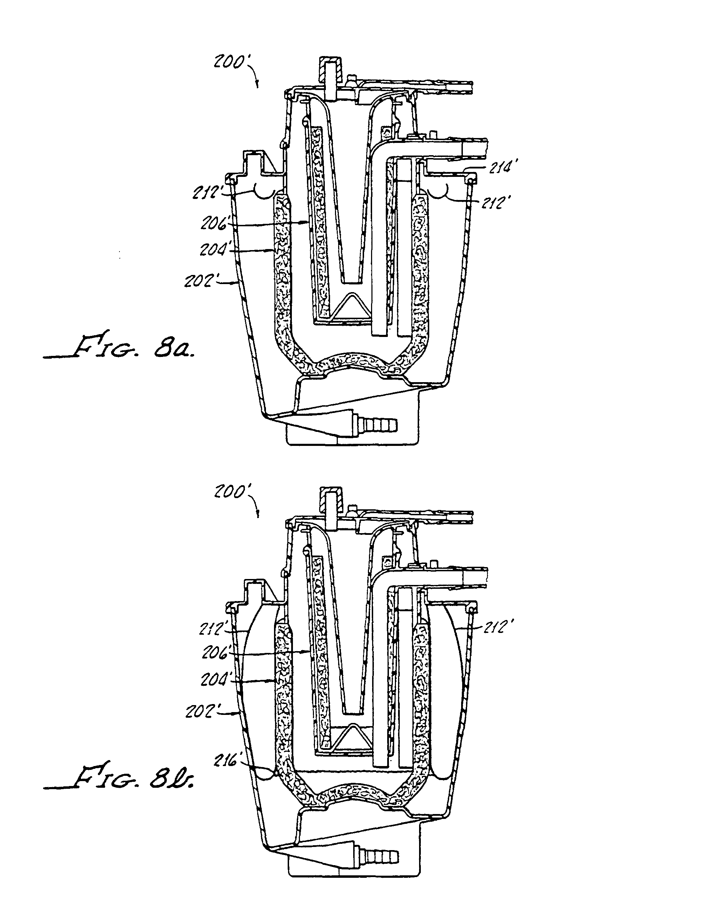

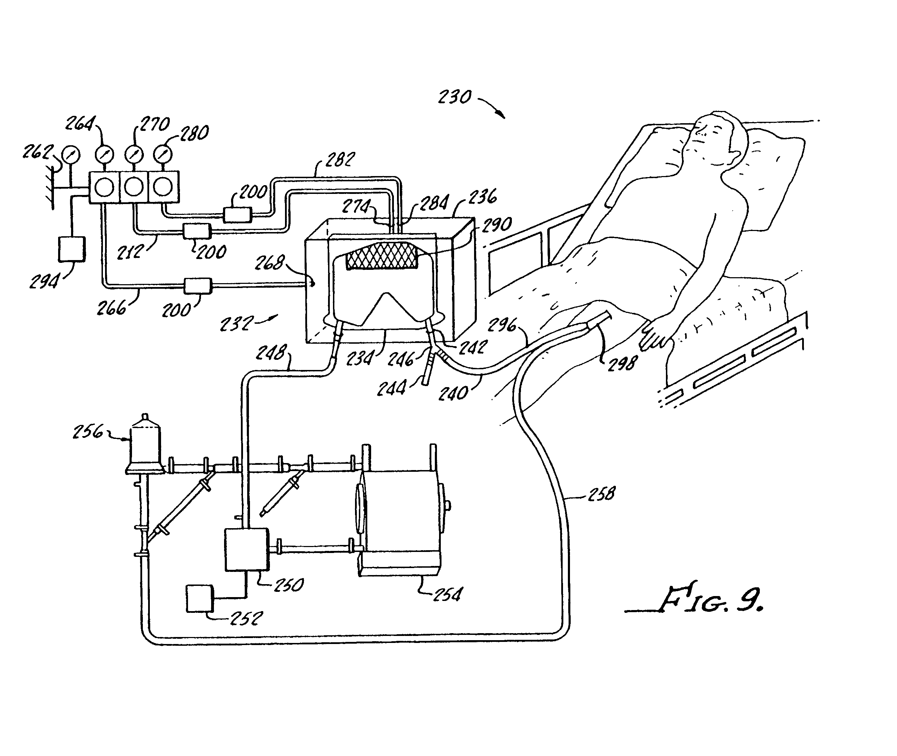

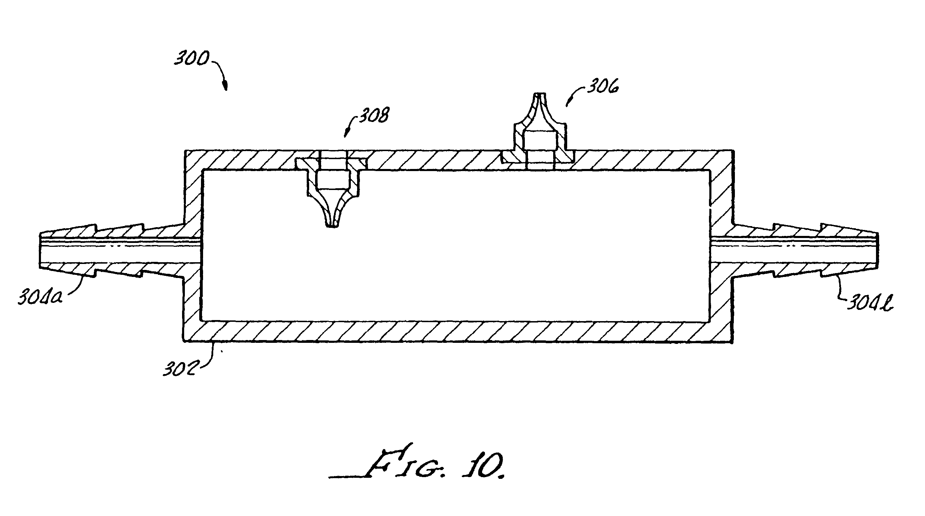

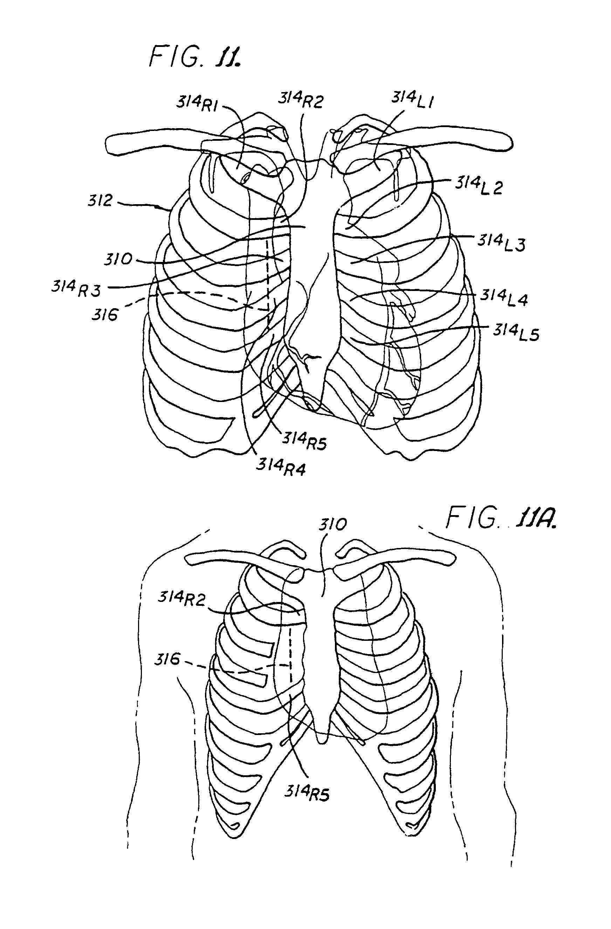

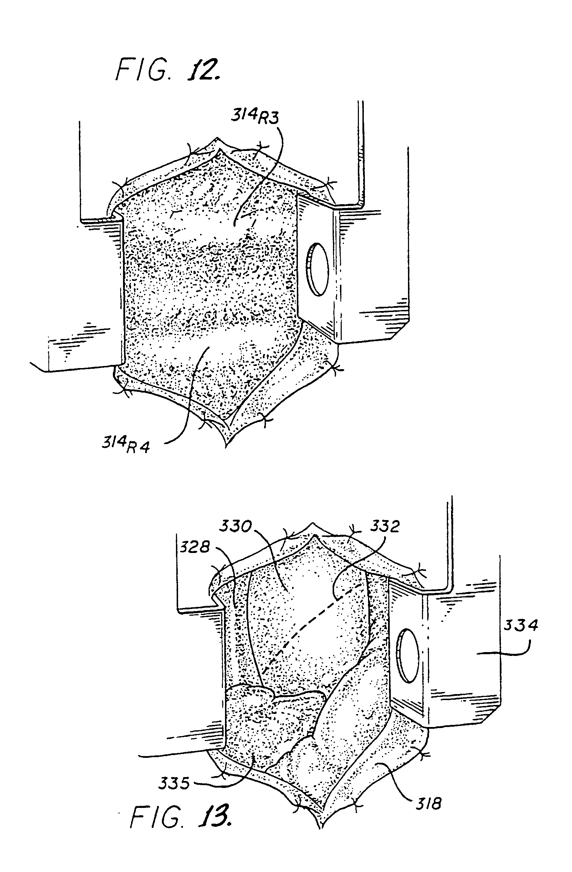

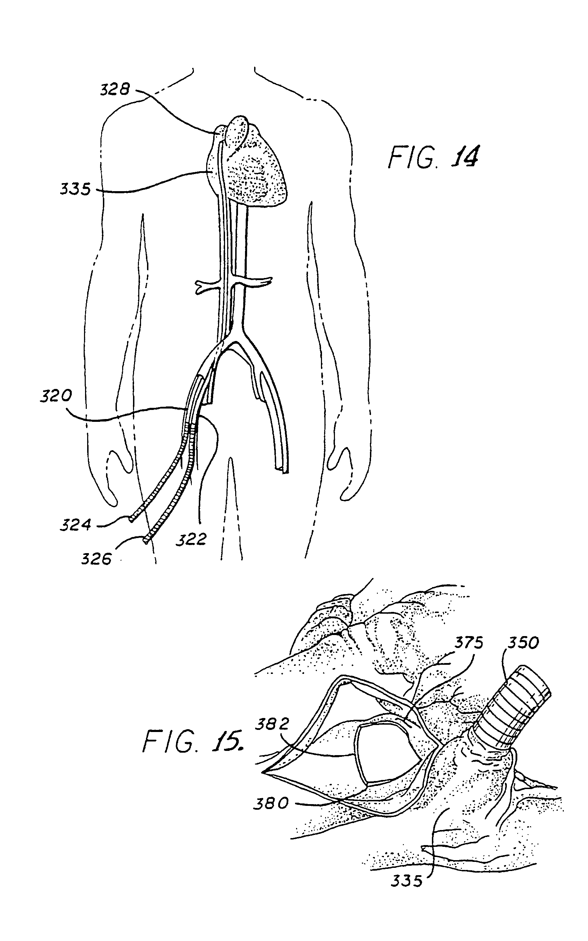

An exemplary vacuum-assisted venous drainage system 10 used in a CPB system is generally illustrated in Figure 1 and preferably includes a sealed reservoir 12 interconnected with a vacuum regulator subassembly 14, a valve subassembly 16, and a vacuum supply line 18 interconnected with a vacuum wall source 20. The reservoir 12 is preferably supplied with blood flow from the patient P via reduced diameter cannulae 34, and may be interconnected with either a heart/lung machine, partially illustrated in Figure 1, or to a combination of components used in the heart/lung machine, such as roller pumps 26, 26a, 26b, 26c, a blood oxygenation and heat exchange unit 28, and a filtration unit 30. A processor or controller 27 interconnected with various sensors and controllers of the system as described below may be provided to regulate the speed of the pumps 26. It will be understood by one of ordinary skill in the art that many of these components are conventional, and readily available from numerous well known sources.

The improved system preferably uses cannulae having diameters of approximately 20 French (Fr) to 28 Fr, but it is anticipated that even smaller diameters may be used. As schematically illustrated in Figure 1, these are either inserted directly into the right atrium RA of the heart to the vena cava, or otherwise as desired. The cannulae 34 may be of a variety of configurations and sizes, but for minimally invasive surgery are preferably selected from a group of cannulas previously used for pediatric or small adult patients. For example, a typical large bore cannula for open heart surgery may have an OD of 36 Fr on its tip, while cannulas for pediatrie or small adult applications have tips of between 18 Fr and 26 Fr. Such small bore cannulae are not presently used for conventional gravity impelled venous drainage in heart surgery on large adults because of their reduced volumetrie flow capacity. With the present vacuum-assisted drainage system 10, the cannulae 34 can be ever smaller in size, which facilitates minimally invasive surgery techniques. In conjunction with the vacuum drainage system 10, modern thin-walled cannulas are preferably used which are fabricated by an extrusion manufacturing process, as opposed to a dipping process. Extruding the venous return cannula enables a thinner walled construction, and associated larger lumen size for any particular outer diameter. Such cannulas can be obtained from Research Medical Inc., of Salt Lake City, Utah, a subsidiary of Baxter International Inc. of Deerfield, Illinois.

The cannulae 34 are interconnected with conventional 9.5mm (3/8 inch) surgical tubing 36, which in turn is interconnected with an inlet port 42 of the reservoir 12. In the preferred embodiment the reservoir 12 is an HSR-4000 Gold hard-shelled venous reservoir that is closed to the atmosphere and has a fixed volume, or is not flexible. The reservoir is available, for example, from Baxter Healthcare Corporation of Irvine, California, a subsidiary of Baxter International Inc. of Deerfield, Illinois.

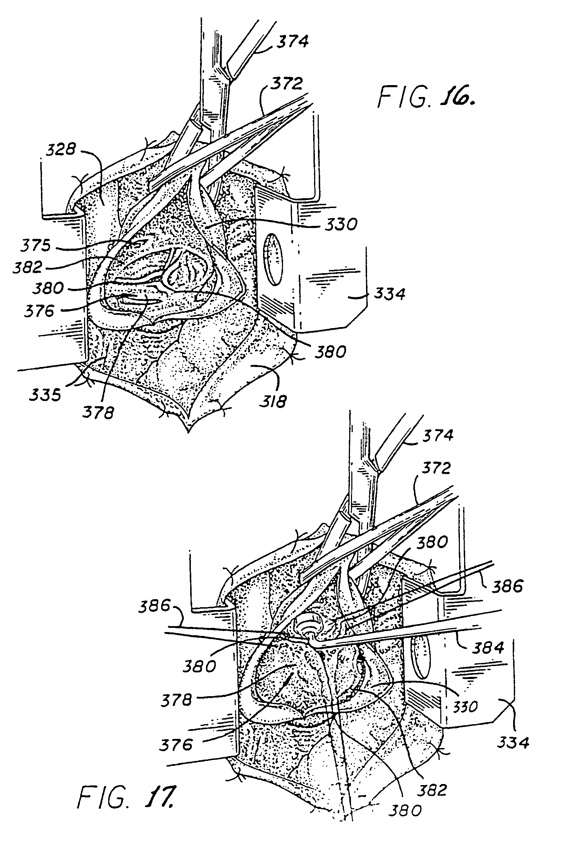

The reservoir 12 includes various inlet and outlet ports described as follows: vacuum inlet 40 indirectly connected to the vacuum supply line 18 and vacuum wall source 20 which supplies a vacuum to the reservoir; the venous blood inlet 42 which receives venous blood flow from the patient via tubing 36, 36a; and a blood outlet 44 which supplies blood from the reservoir to the blood oxygenation and heat exchange unit 28, the blood filtration unit 30 and the patient P, using the roller pump 26. An additional optional venous blood supply line 36b may also be provided, but is shown closed by clamp C. The additional cardiotomy blood inlets 46, 46a may also be used, as in the illustrated embodiment, but may also be sealed using conventional caps or plugs in these connectors. In the alternate embodiment of Figure 5, cardiotomy blood inlet 46' may receive cardiotomy blood via line 49', vent lines 47' and suction line 48'. The vent and suction lines 47, 48 are manually operated by the surgical staff to remove blood from the patient P.

It is noted that where similar or duplicate elements are referred to they will be referred to with an additional alphanumeric designation, and where they are present in an alternate embodiment of the present system, the elements will be referred in with a prime designation. In either case, duplicate elements will not be described in further detail to the extent their performance is substantially similar to the embodiments previously described. For example, the roller pumps illustrated in Figure 1 will be referred to as 26, 26a, 26b, etc., and in Figure 5 as 26', 26a', 26b', etc..

To confirm the vacuum level within the reservoir, negative pressure is monitored prior to entry of blood into the reservoir 12. The conventional negative pressure monitor 38, for example, a digital Series 60000 pressure display monitor available from Medtronic DLP, Inc. of Grand Rapids, Michigan, is positioned to receive blood via tubing 37 from an interconnecting joint 36j intermediate tubing 36 and tubing 36a. A conventional Luer port 39 is also provided at this interconnection so that blood samples may be withdrawn if desired. The preferred negative pressure of blood, which is continuously measured at this point within the system, is approximately -3333 to -9332 Pa (-25 to -70 mmHg).

The vacuum inlet connection 40 to the reservoir 12 is interconnected with a reservoir supply line 50, which is indirectly connected with the vacuum wall source 20. This series of interconnections provides a vacuum to the reservoir, to place the reservoir under negative pressure and enable drainage of venous blood from the patient P through the system. In the preferred embodiment, the vacuum wall source 20 used is the conventionally available source of vacuum supplied to many, if not all U.S., surgical rooms. As previously described, the wall source supplies a vacuum at a constant pressure of between approximately -59995 Pa and -22664 Pa (-450 and -170 mmHg). Vacuum supply line 18 attaches to the wall source 20 via a conventional fitting.

Intermediate the reservoir supply line 50 and the vacuum supply line 18, a vacuum regulator subassembly 14 and valve subassembly 16 are provided. The regulator subassembly 14 includes a vacuum gauge 60 and a vacuum regulator 62. The vacuum gauge 60 is used to monitor the negative pressure level of the system, and is preferably a conventional Duro-United vacuum gauge, with an inlet port 61. The vacuum regulator 62, has a delivery gauge 64 with an on/off lever 66, and an adjustment knob 68 to enable increasing and/or decreasing adjustment of the pressure level as desired. As shown in Figure 3, the vacuum regulator is a conventional general purpose suction regulator available from Nellcor Puritan-Bennett Co., having a first inlet port 69 and a second outlet port 70.

For greater sophistication, the regutator 62 may be controlled by a controller 65 which receives input from sensors in various locations within the system 10. For example, a pressure sensor 67 may be provided in the venous return line 36 to sense overpressure caused by blockage in the line or cannula 34, or other such occlusion. Another sensor 69 may be provided to sense the pressure within the reservoir 12.

A manifold 72 is interconnected between the vacuum gauge inlet port 61, the vacuum regulator first inlet port 69 and the vacuum supply line 18. In the illustrated embodiment of Figure 3, the manifold 72 is a section of hollow steel tubing with first, second and third ports 74, 76, 78, respectively. Threaded fittings connect the manifold 72 with the inlet port 61 of the vacuum gauge 60 at the first port 74, and the first inlet port 69 of the vacuum regulator 62 with the second port 76. There is a friction fit engagement between the manifold 72 and the vacuum supply line 18 at the third port 78. Using this arrangement, the manifold 72 is continuously supplied with negative pressure via the supply line 18. The manifold 72 supplies the vacuum gauge 60 and the vacuum regulator 62. The vacuum gauge 60 provides a reading of the negative pressure level within the system emanating from the wall source 20. Through the vacuum regulator 62, the present system is supplied with negative pressure at the level set using the adjustment knob 68 and the on/off lever 66. In the illustrated embodiment of Figure 3, the manifold 72 is shown clamped within a conventional adjustable support clamp 80. The support clamp is itself mounted on a conventional adjustable horizontal clamp 82. The horizontal clamp 82 clamps to a vertical pole 84 which is secured to the surgery room floor or other fixed equipment. The vertical pole 84 likewise adjustably supports the negative pressure monitor 38.

The reservoir 12 and reservoir supply line 50 are indirectly supplied with negative pressure via the second port 70 of the vacuum regulator 62. Intermediate the vacuum regulator 62 and the reservoir supply line 50 is a conventional vapor trap 86. The vapor trap 86 protects the regulator subassembly from contamination or damage due to vapor return from the direction of the reservoir supply line 50.

The valve subassembly 16 is positioned intermediate the regulator subassembly 14 and the reservoir 12. The valve subassembly 16 includes a conventional check valve 88, which is supplied with negative pressure from the vapor trap 86 via tubing 87. The check valve 88 serves as a safety relief valve, which, when the system negative pressure level reaches -10665 Pa (-80 mmHg), the valve operates to let in room air. Another vapor trap 90 is interconnected with the check valve 88 via tubing 89 further protects the vacuum regulator subassembly 14. The trap 90 may be supported on the roller pump 26, as illustrated in Figure 1, or on other available support structure.

As illustrated in Figure 2, negative pressure is supplied to the reservoir 12 via tubing 50a, an interconnecting joint 50j, tubing 50 and ultimately through the vacuum inlet 40. A manual system disable line 52 of conventional tubing also extends from the interconnecting joint 50j and is provided with a surgical clamp 54. When the system is in "on" condition, supplying negative pressure to the reservoir 12 for drainage of venous blood from rhe patient P, the clamp 54 closes on the tubing 52 as shown in Figure 2. Where the clamp 54 is removed, the system 10 is open to atmosphere, and no vacuum is provided to the reservoir 12. This manual system disable line 52 provides a convenient "on" to enable the system, as well as an immediate shut off, should this become necessary during system operation.

The valve subassembly 16 may also take the form of a combination pressure relief valve and vacuum stabilizer unit 85 (Figure 10). The unit 85 may take a variety of forms, but provides the functions of relieving pressure and stabilizing the vacuum supplied to the reservoir 12 by resisting large changes in the magnitude of the vacuum. One particular embodiment of the combined pressure relief valve in vacuum stabilizer unit 85 is illustrated and described with respect to Figure 10. Of course, the pressure relief valve and vacuum stabilizer may be provided separately and not as a unit.

Prior to operation of the system, the adjustment knob 68 (or controller 65) of the vacuum regulator 62 is used to preset the estimated desired vacuum level. The desired vacuum level is estimated based upon numerous patient characteristics and surgery factors, such as size of the patient, the procedure being performed, the cannulae being used, etc., which are well known to those of ordinary skill in the art, and range between -3333 and -9332 Pa (-25 and -70 mmHg). Once the patient is prepared, the arterial pump 26 of the patient support unit, is then activated. Likewise, the cardioplegia supply pump 26a may be activated when it is desired to supply the patient P with additional blood/fluid components. The vent pump 26b and suction pump 26c may also be activated to remove blood from the patient P as desired.

As shown in Figure 1, cardioplegia fluid is supplied to the cardioplegia supply pump 26a from one or more supply bags 120 (containing either blood or other fluids) via tubing 122. Activation of the pump 26a initiates flow of cardioplegia fluid mixed by the pump 26a through a heat exchange unit 124 having a heating/cooling inlet port 106a, and an outlet port 106b. The unit 124 is supplied with bot or cold fluid, typically water, depending on the temperature change desired, via the inlet port 106a, which fluid is removed via the outlet port 106b. Following appropriate heating or cooling, the cardioplegia fluid is pumped via tubing 122a to the patient P as indicated. The activation of the vent pump 26b and/or suction pump 26c, removes blood from the patient via the hand held devices illustrated or other conventional mechanisms, to the vent line 47 or suction line 48, respectively. The roller pumps 26b, 26c, supply the removed blood to the cardiotomy blood inlets 46, 46a, respectively, via tubing 49, for combination with the direct venous blood flow to the reservoir 12.

Turning again to the further operation of the system 10, when the vacuum regulator "on/off" lever 66 is in the "on" position, and the manual system disable line 52 is clamped in the closed condition, the system is supplied with negative pressure and venous drainage to the reservoir 12 immediately commences without requiring priming of any of the lines 36, 36a, reservoir 12, or oxygenator 28. The system vacuum levels are confirmed on the negative pressure monitor 38, vacuum gauge 60 and delivery gauge 64.

The venous blood flow B supplied to the filtered reservoir 12 is returned to the patient P via pump 26 of the patient support unit, as previously described. In the illustrated embodiment of Figures I and 5, blood exits the reservoir 12, 12' through the blood outlet 44, 44' to, and using, the roller pump 26, 26' and tubing 100, 100'. The blood is then pumped in the direction of the arrows illustrated, via tubing 102, to the oxygenation and heat exchange unit 28 for removal of CO₂ and the addition of oxygen.

As seen in Figure 2, the unit 28 is of a conventional design, with a gas exhaust 104 for CO₂ output, a gas inlet (not illustrated, but positioned adjacent the gas exhaust 104) for oxygen input, and a stainless steel support structure 32. One such oxygenator 28 suitable for use in the illustrated CPB circuit is available under the product name Spiral Gold from Baxter Healthcare Corporation of Irvine California, a subsidiary of Baxter International Inc. of Deerfield, Illinois. The oxygenator unit 28 is supplied with hot or cold water, depending on the temperature change desired, via heating/coolant inlet port 106, and an outlet port (not illustrated). As with the cardioplegia heat exchange unit 124, the hot or cold fluid is provided to the inlet port 106 at a rate of approximately 20 l/min, for appropriate temperature adjustment of the blood or fluid between 10°-37° C. The warmed blood B is then returned to the patient P via outlet 107 and tubing 108, 108a, 108b through the filtration unit 30. Tubing 110 supplies the filtered blood directly to the aorta A via reduced diameter cannulae 34, as illustrated in Figure 1. The filtration unit 30 is conventionally available, and provides a filter of 20 µm pore size for blood passing therethrough. A prime port 130 permits the return of blood, as well as vapor, to the reservoir 12 via prime inlet 134. The present system provides return blood flow to the patient at approximately 7 l/min. In the event additional blood flow is required, or filtration is not required, blood flow may be provided to tubing 110 for direct return to the patient via tubing 108c. The surgical clamp Ca is manually used to determine the desired flow pattern. As further noted in Figure 1, conventional prime ports 130, 132 may be provided from the filtration unit 30 and oxygenation and heat exchange unit 28, respectively, to prime inlets 134, 134a in the reservoir 12 via the tubing indicated. The priming fluid may be provided from supply bags 136 via the tubing as indicated in Figure 1. As shown in Figure 1, conventional priming of the filtration and oxygenation and heat exchange units may be clamped or valved to prevent or permit flow as may be desired. The availability of such return lines to the reservoir 12 permits recirculation of blood flow during use of the system as may be required.

In the embodiments of Figures 1-4, it should be understood that four roller pumps 26, 26a, 26b, 26c are used, as blood from the vent line 47 and suction line 48 is removed from the patient using the positive pressure of roller pumps 26b and 26c, respectively. In the embodiment of Figure 5 of the present system only three pumps are used. The operation of two vent and suction pumps are combined in one pump 26c' to supply blood pumped from the patient to the reservoir 12'. As shown in Figure 5, the present vacuum-assist system is used to indirectly connect a single or multiple vent and/or suction tubing lines supplying blood from the patient P to the pump 26c', under a vacuum. It will be understood by one of ordinary skill that any number of vent lines may be used in the present system during operation of the system.

As illustrated in the preferred embodiment of Figures 5 and 6, each of the vent and suction tubing lines 47', 48', respectively, supply blood from the patient to an intermediate reservoir subassembly 140, supported on an adjustable bracket 82a. The intermediate reservoir subassemblies 140 are under a predetermined desired negative pressure as illustrated, which is -1333 Pa (-10 mmHg) for the vent lines, and -2666 Pa (-20 mmHg) for the suction lines. As illustrated in Figures 5 and 6, between each of the intermediate reservoir subassemblies 140 and the vacuum wall source 20' are elements of the vacuum regulator subassembly 14' substantially as previously described and illustrated. A vacuum gauge 60' monitors system vacuum levels, and individual vacuum regulators 62' for each vacuum line are provided to adjust the negative pressure level as needed. The manifold 72' interconnects each of the respective regulators 62' and the gauge 60'. Vapor traps 86' are additionally used adjacent each of the regulators 62' to protect the regulators from vapor damage.

In addition to the valve subassembly 16' components which are similar to those in Figure 1, the embodiment of Figures 5 and 6 includes additional check valves 88a positioned between the vapor traps and the intermediate reservoir subassemblies 140 to prevent high negative pressure as previously described. Each of the intermediate reservoirs 140 is a hard shelled, sealed unit, preferably including a replaceable liner or bag 146. Due to the use of such liners, the intermediate reservoirs are preferably reusable. Cardiotomy blood is supplied from the vent and suction lines to an inlet 141 of the intermediate reservoir subassemblies 140 under a vacuum, and then flows to the reservoir 12' via the transfer or positive pressure roller pump 26c'. The blood is removed from each of the intermediate reservoirs 140 via reservoir tubing 142 to an outlet 144, which is interconnected with tubing 89a, and by the interconnection illustrated, with tubing 89b.

Other differences illustrated in the embodiment of Figure 5 include the elimination of priming lines to the reservoir 12', as well as the connection of the prime port 132' from the oxygenation and heat exchange unit 28' directly to the input of the cardioplegia pump 26a', at tubing 122', for mixing by the pump 26a'.

Flow rates for venous blood flow both to and from the system using the embodiments illustrated and described with reference to Figures 1-6 are preferably in the range of 0.1 to 7.0 L/min, depending on the procedure used.

### Vacuum-Assisted Venous Drainage Performance

The venous reservoir 12 is characterized by the hard outer shell which is sufficient to withstand any pressures generated within from the wall vacuum 20 and regulator 62. The negative pressure developed within the hard shelled reservoir 12 creates a negative pressure within the venous return line 36, which in turn pulls blood from the vein in which the cannula 34 is placed. Because of the vacuum-assisted venous return suction, the reservoir 12 may be positioned at various locations with respect to the patient P. More specifically, as opposed to prior gravity drain reservoirs, the reservoir 12 may even be positioned above the patient P, although a small elevation below the patient is preferred. Because the reservoir 12 need not be close to the ground, as before, it can be hung adjacent or behind the patient in various locations previously not possible. This greatly increases the flexibility of the operating room set up, and significantly reduces prime volume by reducing the length of tube from the cannula 34 to the reservoir 12.

Volumetric flow results for different cannula sizes, reservoir head heights below the patient, and vacuum magnitudes are presented below in several tables. These results were obtained using a Bentley HSR 4000 reservoir and a container of bovine blood. A roller pump is used to pump the blood from the reservoir back to the container of blood. The blood used had a hematocrit (HCT) level of about 32.5. The head height is the level of the reservoir below the container. The vacuum was connected to the conventional vent port, and all other input ports were plugged. The reservoir level was maintained at 2000 ml and measurements taken at steady state conditions. The range of blood flow through operating bypass systems varies, and a rough estimate for normal adults having a hematocrit level of 25-40 is between 3.5 and 4.5 lpm. It is apparent from these data, therefore, that flow rates sufficient for adult bypass surgery can be obtained with the vacuum-assisted reservoir system of the present invention using cannulas of reduced size. This development promises to revolutionize bypass procedures toward minimally invasive techniques.

**Table 1**

| (1 mmHg = 133,32 Pa) (Cannula Size = 24 Fr) | | | | |
|---|---|---|---|---|
| | | | Pressure Measurements | |
| Head Height (inch) | Vacuum (mmHg) | Pump Flow (lpm) | @ Cannula End (mmHg) | @ Reservoir End (mmHg) |
| 6 | 0 | 1.40 | -21 | 6 |
| 6 | -15 | 2.15 | -35 | -6 |
| 6 | -30 | 2.61 | -49 | -20 |
| 6 | -46 | 3.25 | -62 | -33 |
| 6 | -61 | 3.63 | -75 | -46 |
| 6 | -75 | 4.06 | -88 | -59 |
| | | | | |
| 12 | 0 | 1.75 | -22 | 15 |
| 12 | -15 | 2.39 | -44 | -5 |
| 12 | -30 | 2.80 | -59 | -20 |
| 12 | -45 | 3.50 | -72 | -32 |
| 12 | -61 | 3.98 | -88 | -47 |
| 12 | -76 | 4.36 | -98 | -58 |
| | | | | |
| 18 | 0 | 2.53 | -47 | 3 |
| 18 | -15 | 2.96 | -55 | -5 |
| 18 | -30 | 3.35 | -69 | -18 |
| 18 | -45 | 3.75 | -82 | -31 |
| 18 | -60 | 4.25 | -94 | -44 |
| 18 | -74 | 4.67 | -105 | -55 |

**Table II**

| (1 mm Hg = 133.32 Pa) (Cannula Size = 22 Fr) | | | | |
|---|---|---|---|---|
| | | | Pressure Measurements | |
| Head Height (inch) | Vacuum (mmHg) | Pump Flow (lpm) | @ Cannula End (mmHg) | @ Reservoir End (mmHg) |
| 6 | 0 | 1.10 | -18 | 6 |
| 6 | -15 | 1.53 | -32 | -7 |
| 6 | -30 | 1.91 | -46 | -20 |
| 6 | -45 | 2.31 | -59 | -32 |
| 6 | -60 | 2.63 | -72 | -45 |
| 6 | -75 | 2.98 | -85 | -57 |
| | | | | |
| 12 | 0 | 1.62 | -34 | 3 |
| 12 | -15 | 1.87 | -44 | -6 |
| 12 | -30 | 2.28 | -58 | -19 |
| 12 | -45 | 2.64 | -72 | -33 |
| 12 | -60 | 2.97 | 83 | -45 |
| 12 | -75 | 3.26 | -98 | -58 |
| | | | | |
| 18 | 0 | 1.75 | -41 | 7 |
| 18 | -15 | 2.18 | -55 | -6 |
| 18 | -30 | 2.56 | -70 | -19 |
| 18 | -45 | 2.82 | -82 | -32 |
| 18 | -60 | 3.15 | -95 | -44 |
| 18 | -75 | 3.52 | -109 | -57 |

**Table III**

| (1 mm Hg = 133.32 Pa) (Cannula Size = 20 Fr) | | | | |
|---|---|---|---|---|
| | | | Pressure Measurements | |
| Head Height (inch) | Vacuum (mmHg) | Pump Flow (lpm) | @ Cannula End (mmHg) | @ Reservoir End (mmHg) |
| 6 | 0 | .83 | -32 | 6 |
| 6 | -15 | 1.19 | -46 | -11 |
| 6 | -30 | 1.50 | -59 | -26 |
| 6 | -45 | 1.75 | -73 | -45 |
| 6 | -60 | 2.06 | -86 | -61 |
| 6 | -75 | 2.30 | -99 | -77 |
| | | | | |
| 12 | 0 | 1.20 | -47 | -1 |
| 12 | -15 | 1.48 | -57 | -14 |
| 12 | -30 | 1.79 | -71 | -30 |
| 12 | -45 | 2.03 | -84 | -46 |
| 12 | -60 | 2.31 | -98 | -63 |
| 12 | -75 | 2.53 | -111 | -78 |
| | | | | |
| 18 | 0 | 1.75 | -41 | 7 |
| 18 | -15 | 2.18 | -55 | -6 |
| 18 | -30 | 2.56 | -70 | -19 |
| 18 | -45 | 2.82 | -82 | -32 |
| 18 | -60 | 3.15 | -95 | -44 |
| 18 | -75 | 3.52 | -109 | -57 |

**Table IV**

| (1 mm Hg = 133.32 Pa) (Cannula Size = 18 Fr) | | | | |
|---|---|---|---|---|
| | | | Pressure Measurements | |
| Head Height (inch) | Vacuum (mmHg) | Pump Flow (lpm) | @ Cannula End (mmHg) | @ Reservoir End (mmHg) |
| 6 | 0 | .67 | -34 | 3 |
| 6 | -75 | 1.81 | -104 | -80 |
| | | | | |
| 12 | 0 | .89 | -45 | 3 |
| 12 | -15 | 1.13 | -57 | -11 |
| 12 | -30 | 1.43 | -72 | -29 |
| 12 | -45 | 1.67 | -86 | -45 |
| 12 | -60 | 1.94 | -102 | 65 |
| 12 | -75 | 2.12 | -115 | -80 |
| | | | | |
| 18 | 0 | 1.00 | -55 | 3 |
| 18 | -15 | 1.22 | -70 | -16 |
| 18 | -30 | 1.62 | -84 | -34 |
| 18 | -45 | 1.79 | -96 | -47 |
| 18 | 60 | 2.00 | -112 | -67 |
| 18 | -75 | 2.17 | -127 | -86 |

### Reduced Blood/Air Interface

Hard shelled reservoirs are generally used when larger volumes, reduced resistance to venous return, and accurate blood volumes within the reservoir are needed. These units are typically larger than so-called "soft shell" reservoirs, and since the sides of the reservoir are rigid, flow is less restricted and volume levels at a certain liquid height can be measured and labeled on the reservoir. Hard shelled reservoirs, however, operate partially full so that a blood/air interface at the top of the liquid level exists. In contrast, soft shelled reservoirs are flexible and allow air to be excluded which substantially reduces the blood/air interface. Furthermore, the flexible nature of soft shell reservoirs restrict blood flow and the ability to directly measure the volume of blood within. The present invention provides for a direct visual volumetric measurement of blood while also reducing the blood/air interaction. Reducing blood/air interaction reduces the activation of blood and provides for less blood related complications during bypass surgery.

With reference to Figures 7a and 7b, the present invention provides a vacuum-assisted hard shelled reservoir 200 which includes a mechanism for reducing the blood/air interaction, while also providing the benefits of a direct visual volume measurement. The reservoir 200 may be a modified HSR 4000 reservoir manufactured by Bentley, Inc., or other suitable hard shelled reservoir. In this respect, and as mentioned previously, the reservoir includes an outer canister 202, an inner venous canister 204, and an innermost cardiotomy canister 206. Figure 7a on the left illustrates the reservoir 200 prior to any blood flow therethrough, while Figure 7b shows blood entering through a venous return line 208, and fluid entering the cardiotomy canister 206 through a suction line 210. The reservoir 200 is supplied with a negative pressure, as described above with respect to Figure 1.

To reduce the blood/air interface, a highly flexible, air impermeable membrane 212 is mounted in an upper area of the region between the outer canister 202 and the venous canister 204. In the illustrated embodiment, the membrane 212 comprises an annular tube of highly flexible material with a generatrix or circumferential edge being attached to the underside of the reservoir cap 214. The tubular membrane 212 may be attached by an adhesive, solvent bonding or other expedient methods. The tubular membrane 212 is constructed from a material such as a low durometer polyurethane or silicone, and is sufficiently flexible to expand and fill the inside of the reservoir 200 between the outer canister 202 and the venous canister 204 above a blood surface 216, as seen in Figure 7b. Under atmospheric pressure, the tubular membrane should contain a small amount of air or inert gas within. The vacuum created within the reservoir 200 expands the tubular membrane 212 to fill the space above the blood surface 216. As the vacuum level in the reservoir 200 is varied leading to changes in blood level, the membrane 212 will expand or contract and maintain contact with the surface of the blood without significantly hindering flow. Preferably, the membrane 212 is configured to contact substantially all of the surface of the blood in the annular space outside of the venous canister 204. This essentially limits blood/air contact and associated blood related complications to the spaces within the venous canister 204.

Figures 8a and 8b illustrate another vacuum-assisted hard shelled reservoir 200' which is configured identically to the reservoir 200 described in Figure 7. Common components of the reservoir 200' are thus indicated by a prime. A flexible air impermeable membrane 212' is substituted for the tubular membrane 212 shown in Figure 7a. Instead of being a contained tube, the membrane 212' is formed of a sheet of highly flexible material with its longitudinal edges attached to the underside of the cap 214' to form a U-shape. In all other respects, the flexible membrane 212' acts in the same manner as the earlier described tubular membrane, and expands as in Figure 8b upon initiation of a vacuum to substantially fill the space between the outer canister 202' and the venous canister 204'. Again, this substantially reduces the blood/air interface within the reservoir 200'.

Figures 7a and 7b also illustrate a system for accurately determining the volume of blood within the reservoir. The system comprises an ultrasonic sensor 220 mounted to a lower wall 222 of the canister 202. The sensor 220 is mounted to be directly underneath the space between the outer canister 202 and the venous canister 204. The sensor 220 provides information to a control system 224 which may be in communication with control systems for the vacuum generator, or blood pump, such as the controls 27 and 65 illustrated in Figure 1. The sensor detects the level of blood within the reservoir 200, such as the blood surface 216 in Figure 7b, and sends that information to a processor which is able to compute the volume of blood in the reservoir. Such a sensor is shown and described in U.S. Patent Nos. 5,303,585 and 5,586,085, both to Lichte. Accurate knowledge of the blood level within the reservoir 200 enables rapid response to varying blood flow conditions so that the vacuum may be adjusted to increase or decrease the flow, or for other purposes such as metering an anticoagulant added to the extracorporeal blood to reduce clotting.

### Soft Shell Reservoir Vacuum-Assisted Venous Drainage System

Figure 9 shows a so-called "key hole" CPB procedure 230 utilizing a soft shell reservoir venous drain system 232 including a soft shell reservoir 234 located completely within a rigid housing 236. The reservoir 234 receives venous blood via a venous return line 240 which enters the housing 236 through a sealed aperture 242. A cardiotomy line 244 leading from a cardiotomy filter (not shown) may be joined to the venous return line at a Y-junction 246. A drain line 248 connects the output of the reservoir 234 with a pump 250, which may have a controller 252. The pump 250 sends blood through an oxygenator 254, an arterial filter 256, an arterial line 258, and finally to the patient's arterial system.

In the "key hole" procedure 230 shown, the venous return line 240 and arterial perfusion line 258 join at 296, but remain fluidly separated. The two lines are introduced in parallel into the patient's femoral vein using a dual-stage cannula 298. The venous return line 240 is in communication with a suitable source of venous blood, and the arterial perfusion line 258 is in communication with a location suitable for oxygenated blood perfusion. With this type of surgery only one access incision is needed and the perfusion lines are positioned out of the way of the patient's thorax, where other instruments or probes may be inserted to operate on the heart.
The blood from the venous system is pulled into the reservoir 234 by a negative pressure gradient in the venous return line 240 created by a negative pressure in the reservoir. A source of vacuum 262, such as a wall vacuum, is connected to the interior of the housing 236 via a pressure regulator 264 and vacuum line 266. The vacuum line 266 projects through a sealed fitting 268 into the housing 236. A negative pressure created in the housing 236 tends to inflate the reservoir 234 which in turn, creates a vacuum therein to draw the venous blood from the patient. The reservoir 234 may be of a variety of types, but is preferably one of the following made by Bentley, Inc: BMR-800 Gold or BMR-1900 Gold.

A second pressure regulator 270 communicates with the interior of the reservoir 234 via a conduit 272 entering the housing 236 through a sealed fitting 274. The pressure within the conduit 272 is regulated to maintain a pressure differential between the interior and exterior of the reservoir. This secondary pressure regulation may be used to adjust venous return flow rates.

A third pressure regulator 280 communicates with the interior of the reservoir 234 via a conduit 282 entering the housing 236 through a sealed fitting 284. The pressure within the conduit 282 is regulated to gently pull a suction at the very top of the reservoir 234 to remove any air which may be trapped within. Microbubbles in the blood sometimes combine to form significant air pockets which must be removed before the blood is sent back to the patient. Further, the blood/air interface is detrimental and is preferably eliminated.

As an alternative to the air removal conduit 282, a membrane 290 of sandwiched layers of hydrophobic and hydrophilic materials may be formed into the upper wall of the reservoir 234. This membrane 290 would facilitate passive venting of air from the reservoir 234 due to the vacuum generated within the housing 236.

As with the earlier described hard shell reservoir embodiment, various sensors may be positioned around the system 230 for monitoring pressures, flows, temperatures, blood levels, etc. A microprocessor 294 may be provided to control the three pressure regulators 264, 270, and 280 and enhance the efficiency of the system. The microprocessor 294 may also be connected to the pump controller 252, oxygenator 254, or other device such as a heat exchanger (not shown).

### Pressure Relief Valve/Vacuum Stabilizer

As seen in Figure 9, each of the conduits 266, 272 and 282 between the vacuum regulators and housing 236 include a pressure relief and vacuum stabilizer unit 300, a preferred form of which is illustrated in Figure 10. The unit 300 comprises a chamber 302 open at opposite ends to nipples 304a and 304b. The nipples 304a,b are used to connect the units 300 in series in one of the conduits 266, 272 and 282. The unit 300 further includes a pressure relief valve 306 and a vacuum stabilizing valve 308. The pressure relief valve 306 cracks open at a very low pressure differential threshold and bleeds air out of the line, in response to buildup of positive pressure. The vacuum stabilizing valve 308 continuously bleeds air into the system at a flow rate proportional to the level of vacuum to be maintained by the particular pressure regulator 264, 270 or 280. When the level of vacuum increases within the system to a predetermined threshold value, the vacuum stabilizing valve 308 yields (the opening increases) proportionately to allow an increased amount of air to bleed into the system, thereby compensating for the increased level of vacuum. This helps reduce large swings in vacuum, thus the stabilizing effect. In a preferred form, both the pressure relief valve 306 and a vacuum stabilizing valve 308 are conventional duckbill valves chosen for the desired pressure threshold. This greatly reduces the cost of the system.

### Flow Control

The pressure differential created by the application of a negative pressure at the reservoir end of the venous return line can be regulated to enable control over venous return, independent of the relative positioning of the reservoir to the operating table. As mentioned above, various sensors may be positioned at critical locations for measuring pressure, temperature, flow rates, blood levels, etc. The sensors may be connected to a control system with output to a number of actuators such as the vacuum regulators, circulation pump, secondary regulators, heat exchangers, etc. Certain variables can be sensed and/or controlled with appropriate feedback loops, preferably coded into a programmable microprocessor. These variables include the amount of vacuum applied to the reservoir (or housing around the reservoir), the pump speed or pulse rate, the threshold level of vacuum allowed in the venous return line, and the reservoir minimum and maximum volumes. Such a sophisticated control system would result in reduced hemodilution (prime volume) by enabling reduced cannula sizes, reduced tubing sizes and lengths, accurate control of venous drainage, and enhanced control of arterial return. In addition, benefits gained to perfusion control include positive control of venous drainage and arterial perfusion, and microprocessor control of flow rates. In addition, the microprocessor could be adapted to measure and control circuit temperature and oxygenation. With adequate fail-safes built into the system, the traditional role of the perfusionist is greatly reduced.

### Pediatric Applications

Although cannula sizes for older children and teens range from 18-26 Fr, smaller cannulas are often used for infants and newborns. For example, Research Medical, Inc. (RMI), of Salt Lake City, Utah, provides a line of cannulas down to 8 Fr in size. French (Fr) is a term for the outside diameter (OD) of the cannula, and the conversion to metric is: 1 mm = π Fr. Thus, an 8 Fr cannula has an OD of 2.54 mm. The bore size of a particular Fr cannula will depend on the cannula wall thickness. As mentioned above, RMI has developed an extrusion process which brings the wall thickness of an 18 Fr cannula down to .018 inch (.457 mm) from between .022-.027 inch (.559-.686 mm) for earlier designs fabricated by conventional dipping methods. An 18 Fr cannula has an OD of 5.73 mm. With a wall thickness of .457 mm, the ID is 4.816 mm. Conventional 18 Fr cannulas would have a maximum ID of 4.612 mm. The increase in the cross-sectional flow area through extruded cannulas is thus 9%. This increase, in combination with drawing a negative pressure in the cannula, greatly facilitates the use of smaller and smaller cannulas. It should be emphasized that cannulas smaller than the currently available 8 Fr size may become viable for neonatal care, for example, with the vacuum-assisted drainage and thin walled cannulas. In other words, the benefits of the present invention will be realized by patients of all sizes. Moreover, the reduction in extracorporeal blood prime volume which is realized by locating the reservoir closer to the vein is most significant for neonatals and infants, who have a relatively lesser amount of blood in their vasculatures. Neonatals, for example, may only require a blood flow through the CPB circuit of less than 1 lpm.

### Benefits to Vacuum-Assisted Venous Drainage

The present invention is expected to achieve the following benefits for conventional cardiopulmonary bypass surgery:
1. Venous return flow rates will no longer depend on the physical location of the reservoir with respect to the operating table, providing opportunities for miniaturization of the entire CPB circuit.
2. A miniaturization of the CPB circuit would lead to minimizing blood contact with foreign surfaces thereby reducing complications associated with immune response such as platelet depletion, complement activation and leukocyte activation.
3. Since venous flow is initiated by the application of negative pressure, the venous return line need not be primed. This leads to a substantial reduction in priming volume of the CPB circuit, resulting in reduced hemo-dilution of the patient. This aids in the recovery of the patients physiological status after surgery.
4. Surgeon acceptance of the use of vacuum for venous return, could lead to suction systems taking the place of roller pumps in other applications. This potentially could lead to elimination of roller pumps that support the sucker, sump and vent functions that fall under the responsibility of the perfusionist. This would result in minimizing blood trauma caused by roller pumps. Additionally, this would also free-up valuable floor space in the vicinity of the operating table.

In addition, the present invention is expected to achieve the following benefits for Minimally Invasive Surgery :
1. Vacuum-assist will help achieve higher flow rates through smaller cannulae. This will allow the use of smaller cannulae thereby providing the surgeon greater access to the site of the surgery. This could also potentially eliminate the need for larger size cannulae.
2. MICS techniques, such as the Key Hole concept, use the femoral vein as access port for CPB circuitry. This technique imposes stringent limitations on the cross-section and length of the catheter / cannula used for venous return. Vacuum-assist can replace centrifugal pumps to enhance venous drainage in these applications.

### Minimally Invasive Surgery Techniques

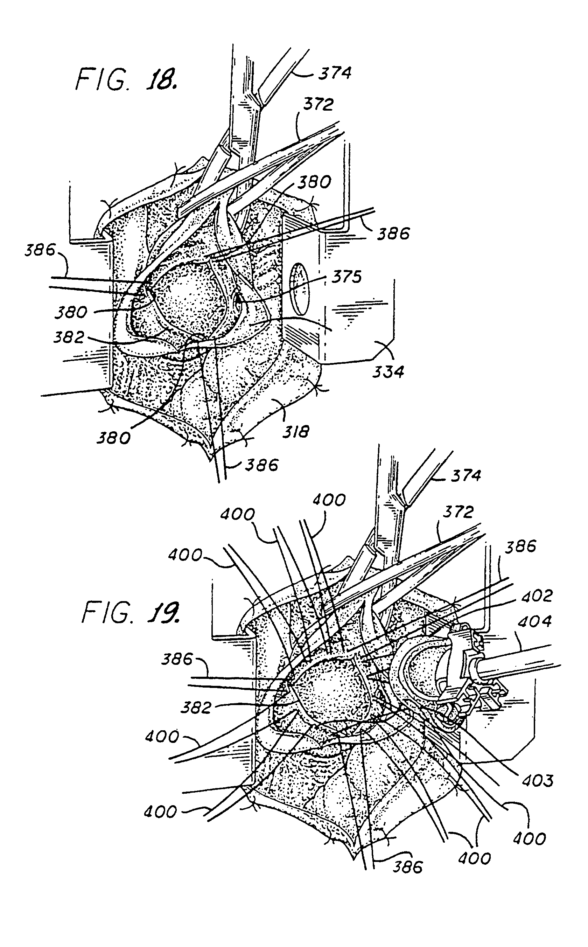

Referring now to Figure 11, in a typical human, a sternum 310, a planary bone structure centrally disposed in the chest, is connected to a plurality of ribs 312 by respective costal cartilages 314_{R1}, 314_{R2}, 314_{R3}, 314_{R4}, 314_{R5}, and 314_{L1}, 314_{L2}, 314_{L3}, 314_{L4}, 314_{L5}. The heart and great vessels are located within a tissue sac (pericardium), located beneath the sternum, extending laterally under the costal cartilages and ribs, with the aorta disposed in part underlying the second and third right costal cartilages 314_{R2} and 314_{R3} and a portion of the right coronary artery located generally underlying the vicinity of the fourth and fifth right costal cartilages 314_{R4} and 314_{R5}.

In accordance with one aspect of the present invention, it has been determined that a surgery on portions of the heart and great vessels located between a point approximately three centimeters above the supra annular ridge and the mid-ventricular cavity of the heart, can be effected with minimal invasion, and without a median sternotomy or other gross thoracotomy. For example, as illustrated in Figure 11A, the surgeon makes a relatively short parastemal incision 316 extending across a predetermined number of costal cartilages, e.g., as well as a right parastemal incision extending from the lower edge of the second costal cartilage 314_{R2} to the superior edge of the fifth costal cartilage 314_{R5} and removes one or more costal cartilages, e.g., the third and fourth costal cartilages, 314_{R3} and 314_{R4}. It has been determined that over a period of time the chest wall in the area of the resected cartilages becomes stable secondary to scarring of the remaining tissue. In effect, scar tissue resulting from the procedure functionally replaces the excised cartilage, providing a relatively rigid chest wall.

This procedure can be readily employed to perform operations on structures located on portions of the heart and great vessels located between a point approximately three centimeters above the supra annular ridge and the mid-ventricular cavity. As will be more fully described, the procedure is of particular utility with respect to surgery to repair or replace the aortic valve. Further, in some instances, the minimally invasive approach of the present invention can be employed to effect a variety of other operations, such as, for example, septal myectomy (excision of a portion of the muscle just below the aortic valve to correct an obstruction to the outflow of the heart); closure of a ventricular septal defect (e.g., a congenital hole in the heart); and correction of aneurysms.

The minimally invasive approach of the present invention is particularly advantageous as compared to a median sternotomy. In addition to decreased trauma to the patient, and the attendant benefits, the minimally invasive technique provides additional advantages in the event of repeat surgery. Since the pericardial sac underlying the sternum is opened under the sternum in a median stemotomy, after the procedure the heart has a tendency to adhere to the sternum. This can be problematical in the event of a subsequent procedure; there is a risk of cutting into the heart when sawing through the sternum during the subsequent operation. In contradistinction, in the procedure according to the present invention, the pericardium underlying the sternum remains intact, normal tissue is retained between the sternum and the heart and there is no risk of the heart adhering to the sternum. A series of operations are relatively common in connection with correction of congenital heart disease.

As noted above, the minimally invasive approach of the present invention is of particular utility with respect to surgery to repair or replace the aortic valve. Specifically, in the context of exemplary surgery to replace an aortic valve, the patient is anesthetized and intubated, and placed supine on the operating room table. Preferably, defibrillator pads are placed on the patient's back and anterior left chest, and a transesophageal echocardiography probe is placed to access the etiology of the aortic valve disease and to assist in removing air from the heart after completion of the operation.

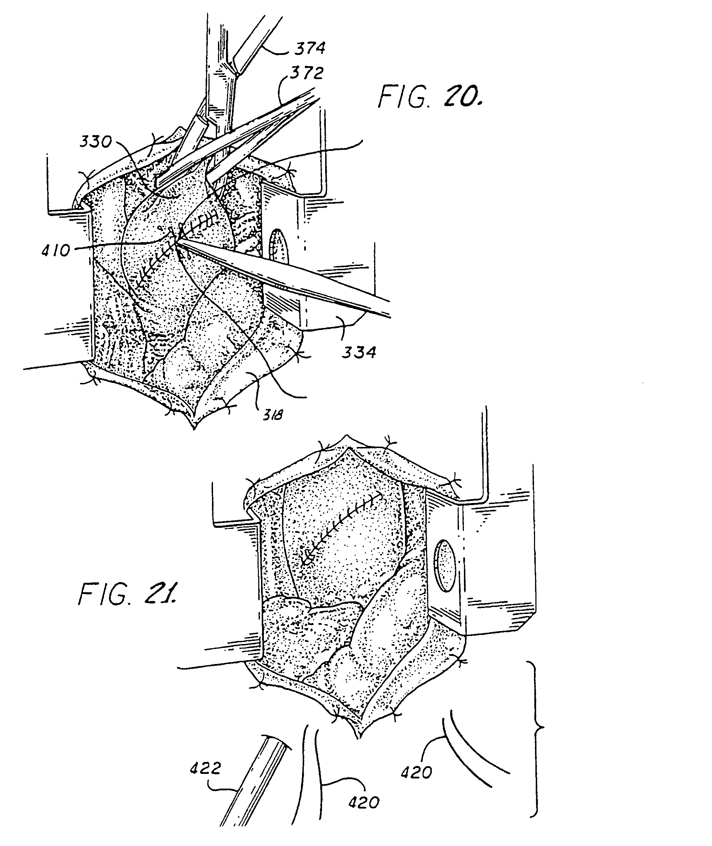

Referring to Figures 11 and 11A, a right parasternal incision is made extending from the lower edge of the second costal cartilage 314_{R2} to the superior edge of the fifth costal cartilage 314_{R5}. The pectoral major muscle is divided, exposing the second, third, and fourth intercostal spaces, and the third and fourth costal cartilages 314_{R3} and 314_{R4} as shown in Figure 12. The third and fourth costal cartilages 314_{R3} and 314_{R4} are totally excised (Figure 11A). The right internal thoracic artery is ligated just below the second costal cartilage 314_{R2} and just above the fifth costal cartilage 314_{R5}. Intercostal muscles and pleura are incised lateral to the edge of the sternum, entering the right pleural cavity. As shown in Figure 13, the pericardium 318 is then incised, exposing the ascending aorta 330, and is stitched back. The incision is held open using a conventional chest retractor 334.

A cardiopulmonary by-pass is then established. Referring now to Figure 14, a common femoral artery 320 and vein 322 are exposed and, after infusion of an anti-coagulant, i.e., heparinization, are cannulated. Catheters 324 and 326 are placed in femoral artery 320 and in femoral vein 322, respectively. Adequate venous drainage may be obtained by utilizing a long venous cannula 326 disposed so that the tip of the cannula passes through the right atrium 335 and preferably into the superior vena cava 328 (Fig. 13). Alternatively, as illustrated in Figure 15, venous return can be effected by introducing an appropriate catheter 350 into the right atrial appendage 335. (The anatomy depicted in Figure 15 illustrates the results of additional steps in the procedure, as will be explained). Catheters 324 and 326 direct the blood to a conventional heart-lung machine (not shown) which oxygenates the blood and pumps it back under pressure to the patient.

Referring to Figure 16, after catheters 324 and 326 are placed, the heart is excluded from circulation: aorta 330 is suitably encircled with umbilical tape 372 and the ascending aorta 330 cross clamped with a right angle clamp 374.

With continued reference to Figure 16, the aorta is then incised (along line 332, Fig. 13) to expose the coronary ostia 375 and the aortic valve 376. Aortic valve 376 includes a plurality, typically three, of leaflets (valve cusps) 378, joined at respective commissures 380, and surrounded by a relatively fibrous aortic annulus 382.

Cardiac function is arrested by, e.g., administering cardioplegia into the ascending aorta. Referring now to Figure 17, after performing the aortatomy, a suitable cardioplegia is introduced into the left coronary artery. Preferably, a suitable cardioplegia fluid, such as a cold potassium solution is infused through a catheter 394 inserted in coronary ostia 375. Sutures 386 are the suitably placed just above each commissure 380, and clamped under tension to a drape (not shown) surrounding the operating site. This elevates the aortic root (e.g., aortic annulus 382) into the operative field.

Aortic valve 376 is then either repaired or replaced. For example, referring to Figures 18 and 19, where a valve replacement is effected, valve cusps 378 are excised, leaving aortic annulus 382 (Figure 18; see also Figure 15). A multiplicity of sutures 400 are then placed through aortic annulus 382 about the periphery of the void left by excision of the valve cusps 378 (Figure 17). Sutures 400 are then employed to secure a suitable replacement valve 402. Replacement valve 402 may be, e.g., a bioprosthesis (cusps formed from animal tissue coupled to a suitable peripheral sewing ring, formed of e.g., polyester velour), a mechanical prosthesis (cusps formed from e.g., pyrolytic carbon with a suitable peripheral sewing ring 403, formed of e.g., polyester velour), or a homograft (e.g., formed from human tissue which was frozen in liquid nitrogen, then thawed). Attachment of the bioprosthesis and mechanical prosthesis replacement valves are suitably facilitated using a conventional insertion tool 404. Replacement valve 402 is typically attached to aortic annulus 382 by passing sutures 400 through sewing ring 403 of the replacement. A vent is intermittently placed into the left ventricle through the aortic annulus as needed.

At the completion of the repair or replacement, the aortatomy is closed with sutures 400, as shown in Figure 20. Air is then removed from the heart through the aorta with the assistance of the transesophageal echocardiography probe; all air bubbles are preferably removed from the heart by removing clamp 374 to restore blood flow, and inflating the lungs, until blood flows through sutures 410, then tightening the sutures.

Referring to Figure 21, temporary pacemaker leads 420, 422 are placed on the atrium and on the ventricle to facilitate temporary pacing should it be necessary. The patient is weaned from cardiopulmonary bypass, the femoral vessels are decannulated and repaired, conventional right-sided pleural chest tubes 422 are placed, and the femoral and right parastemal incisions are closed, suitably by reapproximating the muscle, subcutaneous tissue and skin, in layers.

In another aspect of the present invention, a similar incision as that described above with reference to Figures 11, 11A and 12, can be used in performing surgery to repair or replace a mitral valve. More specifically, referring to Figures 11A and 12, a parasternal incision approximately 10cm in length is made over the third and fourth intercostal cartilages 314_{R3} and 314_{R4}. The pectoralis major muscle is then divided longitudinally, exposing the third and fourth cartilages 314_{R3}, 314_{R4}. The cartilages 314_{R3}, 314_{R4} are completely resected and the internal thoracic artery (not shown) is then ligated and divided. The pericardium 318 is opened and suspended under tension to the drapes of the patient.

Referring to Figure 22, the resulting wound provides access into the chest cavity and particularly exposes the first portion of the ascending aorta 330, the superior vena cava 328 and the right atrium 336. The wound also provides access for making a planned incision 450 into the right atrium 336.

Referring to Figure 23, prior to making the incision 450 into the right atrium 336, the patient must be cannulated so that the heart may be bypassed from blood flow during the surgery on the heart. In that connection, a first cannula 452 is inserted directly into the superior vena cava 328. A second cannula 510 (Figure 33) may be inserted into the inferior vena cava, either via the right atrium 336 or via a venous cannula introduced through a femoral vein as known in the art. Arterial return is established by a third cannula 506 which may be inserted either directly into the ascending aorta 330 as shown in Figure 33 or through a femoral artery as depicted in Figure 14.

The cannulation configuration for heart bypass will be dictated in large part by patient anatomy and physiology particularly with regard to the size and placement of the heart within the chest cavity, and the resulting effect of that anatomy and physiology on the incision exposure. It is desirable, however, to achieve as much of the bypass cannulation as possible through the primary incision so as to reduce the number of incisions otherwise made in the patient for peripheral cannulation as shown in Figure 24.

Once cannulation is complete, a cross clamp 460 is applied to the ascending aorta 330 as shown in Figure 24 to occlude blood flow. Antegrade cardioplegia is then applied directly into the ascending aorta proximal of the clamp via a cardioplegia catheter 462. Bypass is established and then the heart progressively diminishes its beating activity until it ceases beating altogether.

Referring to Figures 23A and 23B, it is appreciated that an aortic occlusion balloon could alternatively be used to block the ascending aorta for establishing bypass. In particular, an aortic occlusion balloon catheter 461 could be introduced either through the femoral artery, as shown in Figure 23A, the sub-clavian artery as shown in Figure 23B or other vessel in a manner to position the balloon between the coronary ostia and the brachycephalic artery of the ascending aorta. Occlusion is achieved by inflating the balloon so that the balloon contacts the internal wall of the aorta and thereby blocks blood flow in the aorta. Cardioplegia may then be introduced into the coronary ostia either directly through the aorta as previously described or through a cardioplegia lumen extending to a distal end of the aortic balloon catheter.

With further reference to Fig. 23B, it is appreciated that under certain circumstances, the method of the present invention can be performed using a retrograde application of cardioplegia. The retrograde cardioplegia catheter placed in the coronary ostia through the jugular vein and the right atrium. It is further appreciated that the type of cardioplegia used, whether introduced antegrade or retrograde, will often be dictated by the anatomy and physiology of the patient or by the preference of the physician.

Once bypass is established, the incision 450 into the right atrium 336 is made and the tissue draped back to expose the coronary sinus 466 and intraarterial septum 464 (Figure 23). Additional cardioplegia is introduced, as necessary, in a retrograde fashion into the coronary sinus 466 with a retrograde cardioplegia catheter 468. The retrograde cardioplegia catheter 468 can be either a conventional retrograde catheter or an occluding balloon catheter to ensure proper introduction of the cardioplegia without leakage. The stage is then set to cut the intra-atrial septum 464 along an incision line 470 and thereby expose the dome of the left atrium.

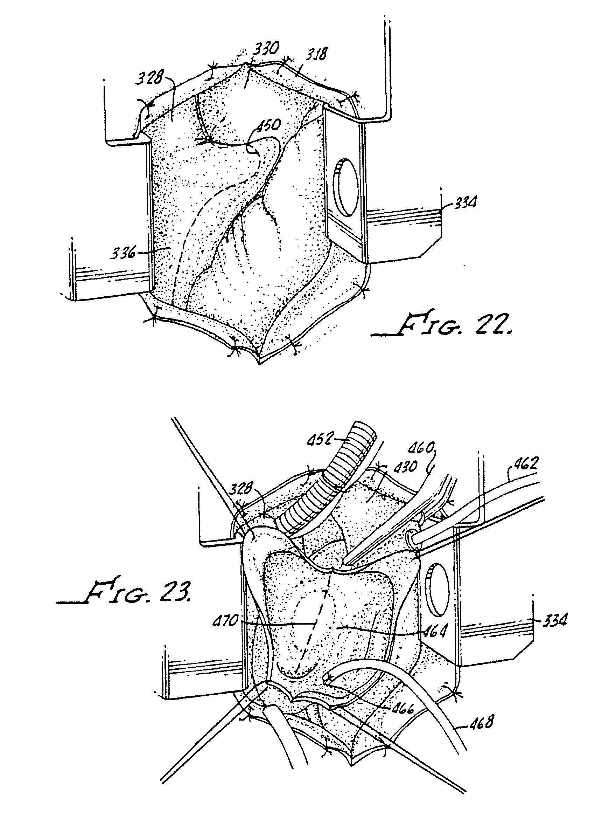

Referring to Figures 24 and 25, the incision 470 is made in the intra-atrial septum 464 starting at the foramen ovale and extending inferiorly and superiorly into the dome of the left atrium. Hand-held refractors 472, 474 are then inserted into the superior and inferior portions of the left atrium, respectively, and used to pull the atrial tissue back and expose the mitral valve 476. Additionally, downward traction may be applied on the posterior lateral left atrial wall 478 to provide better exposure to the mitral valve 476. Referring to Figures 25A and 25B, a deformable retractor 477, which may be manipulated into a shape that grasps the tissue but does not obstruct the surgical field, may be used to provide the downward traction on the posterior lateral left atrial wall 478. In addition, to further expose the surgical field, a flexible and resilient ring member 479 may be inserted into the field between the valve 476 and the left atrial wall. After the ring member is inserted, the ring 479 expands to facilitate lifting the tissue away from the valve area requiring surgery. The mitral valve 476 being fully exposed after achieving the above-described configuration, repair or replacement of the valve 476 may then be achieved in the conventional manner. By way of example only, the procedure for completing the surgical method after repair of a mitral valve is hereinafter described.

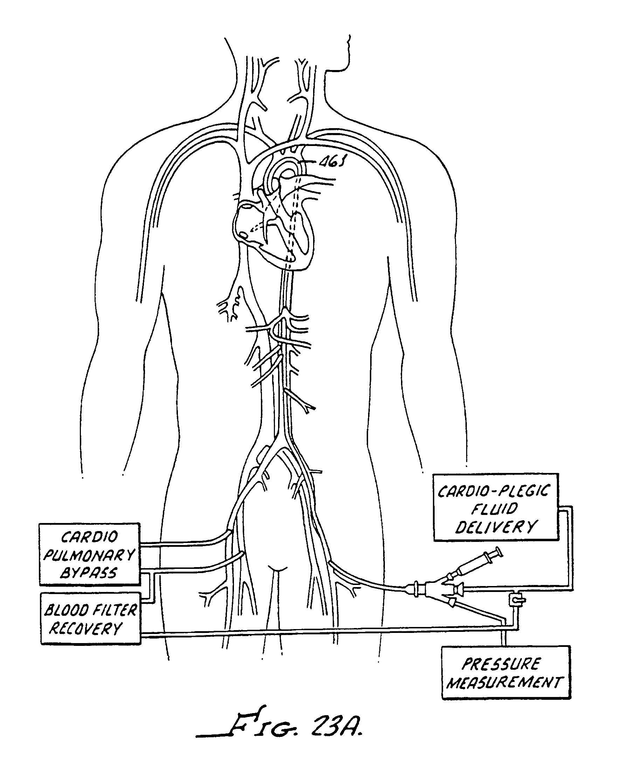

Referring to Fig. 26, after repair of the mitral valve 476, an annuloplasty is performed. In particular, horizontal mattress sutures 480 of multi-filament 2-0 are placed around the annulus of the valve beginning with the fibrous trigone 482 and proceeding around the posterior annulus of the opposite fibrous trigone 484. The sutures 480 are then passed through the annuloplasty band 486 which is attached to a band holder or stent 488.

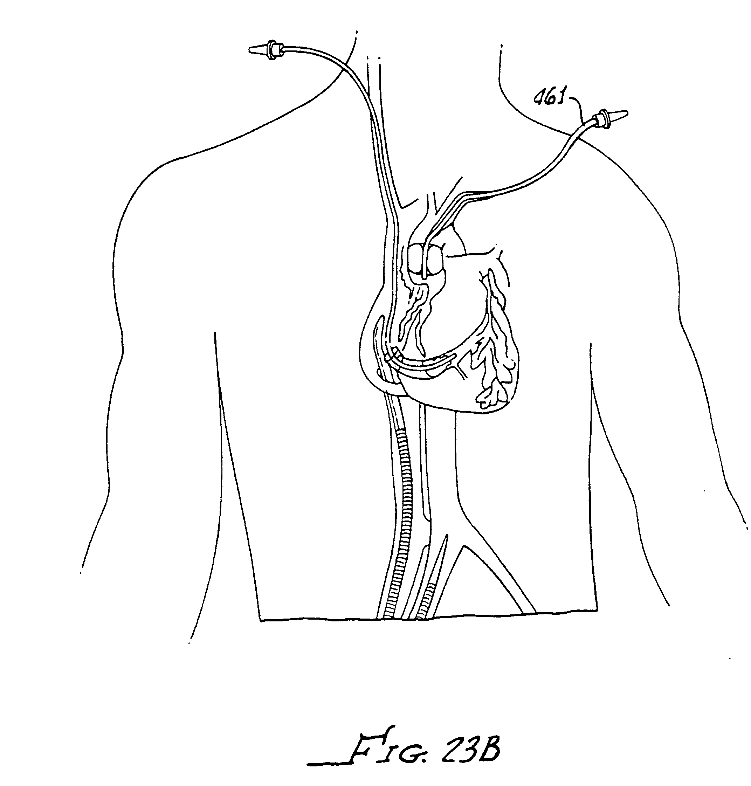

Referring to Figures 27 and 28, once placement of the sutures is complete, the handle 490 of the stent 488 is released and the stent 488 with the annuloplasty band 486 is guided into position proximal to the mitral valve 476. The sutures 480 are tightened and tied down thereby securing the annuloplasty band 486 into place. The stent 488 is then released and removed from the band 486 thus leaving the repaired valve 476.

It is appreciated that the use of other types of annuloplasty rings are contemplated in the just-described surgery. For example, annuloplasty rings that requires suturing around the entire periphery of the ring (e.g., a Carpentier ring or a Duran ring) may be used without departure from the invention.

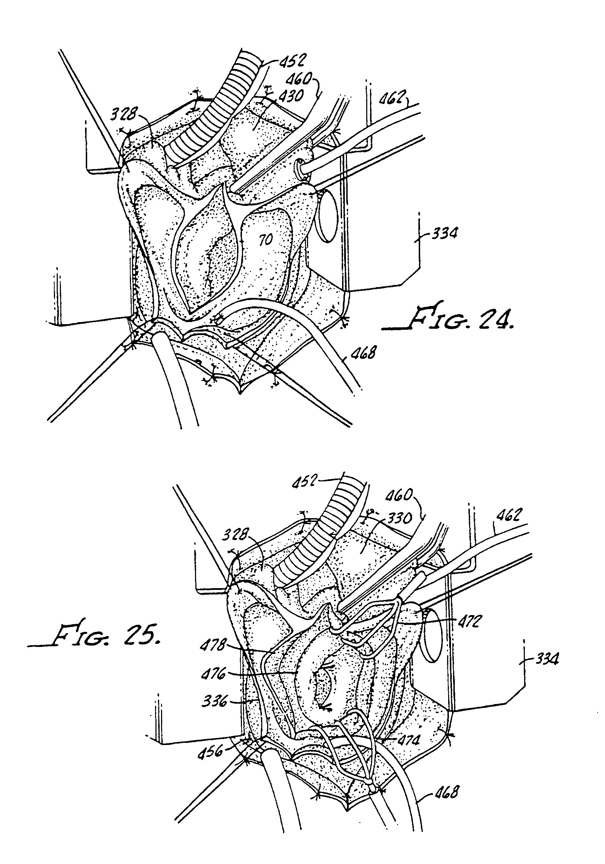

Referring to Figure 29, the incision 470 into the interatrial septum is sutured 492 back together using continuous 4-0 Prolene or other suitable suture material. Attempts are made to remove all air from the left atrium and then the sutures 492 are tightened and tied down.

De-airing of the left ventricle is also effected at this time. In that connection, just prior to release of the aortic clamp 460, gentle suction may be applied on the cardioplegia cannula 462 in the ascending aorta 330. Weaning from the cardiopulmonary bypass is then initiated. The retrograde cardioplegia cannula 468 is removed as is the aortic clamp 462, thereby restoring blood flow. The lungs are then inflated until blood flows through the sutures 492. Suction through the cardioplegia cannula may continue as needed after the aortic clamp 462 is removed.

Referring to Figure 30, the incision 450 in the right atrium is also closed using continuous 4-0 Prolene or other suitable suture material. Simultaneously, the heart is observed to ensure a return to normal cardiac function and to ensure the absence of air bubbles within the heart chambers. If the heart function returns properly, the cannulae are removed and the incisions from the cannulae placement are repaired as needed and sutured shut.

Four pacemaker wires 420 are placed percutaneously through the chest onto the atrium and the ventricle to facilitate temporary pacing should it be necessary. Conventional pleural chest tubes as depicted in Figure 21 may also be placed in the chest. The wound is then closed by suitably reapproximating the muscle, subcutaneous tissue and the skin, in layers.

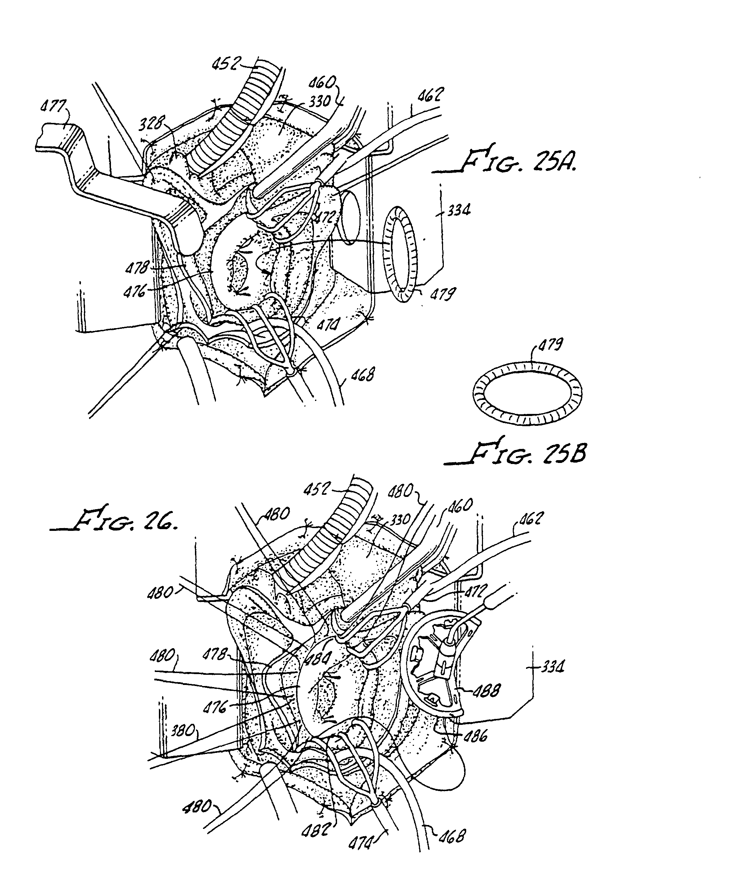

Referring to Figure 31, in another approach to minimally invasive surgery in accordance with the present invention, the patient is anesthetized in the supine position and intubated. Defibrillator patches (not shown) are placed on the patient's back and anterior left chest wall. A transesophageal cardiography probe (not shown) is placed to assess the etiology of the tissue requiring surgery, which by way of example only, is the aortic valve in this embodiment. The cardiography probe is also useful to remove air from the heart prior to completion of the surgery.

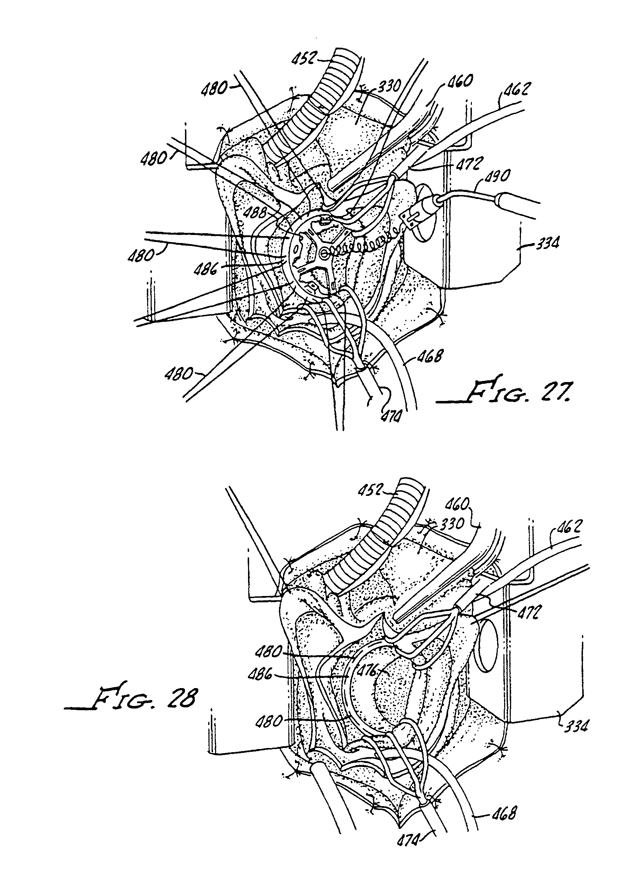

Referring to Figures 31 and 32, a 10 cm transverse incision 500 is made over the second intercostal space. In certain circumstances, it may be appropriate to make the incision over the third intercostal space, depending on the location of the targeted surgical area. The subcutaneous tissue and pectoralis muscles are divided. The internal thoracic artery (not shown) is ligated and divided bilaterally. The tissue is retracted and draped back to better expose the surgical area. A sternal saw (not shown) is then used to divide the sternum 504 transversely in alignment with the original incision 500. A retractor 334, such as a Finochietto retractor, is placed between the two bisected portions of the sternum 504 and the sternum opened. The separation of the sternum 504 and the subsequent cutting and retracting of the pericardium exposes the entire ascending aorta 330, the superior vena cava 328 and the tip of the right atrial appendage 336.

Referring to Figure 33, the patient is cannulated for heart bypass by inserting an arterial return cannula 506 directly into the ascending aorta 330 and a venous drain cannula 508 into the superior vena cava 328. A venous drain cannula 510 is also inserted into the inferior vena cava through a percutaneous incision 512 proximal to the original incision opening.

Once cannulation is completed, the aorta 330 is occluded at a position proximal, of the brachycephalic artery and distal of the coronary ostia 522 with a cross-clamp 516 and bypass of blood flow around the heart is initiated. As discussed previously, an aortic occlusion balloon inserted through a femoral artery or sub-clavian artery could also be used to block the aorta 330. A transverse incision 518 is made in the aorta 330 from a position proximal to the clamp 516 into the noncoronary cusp 520, which incision exposes the coronary ostia 522 and the aortic valve 524.

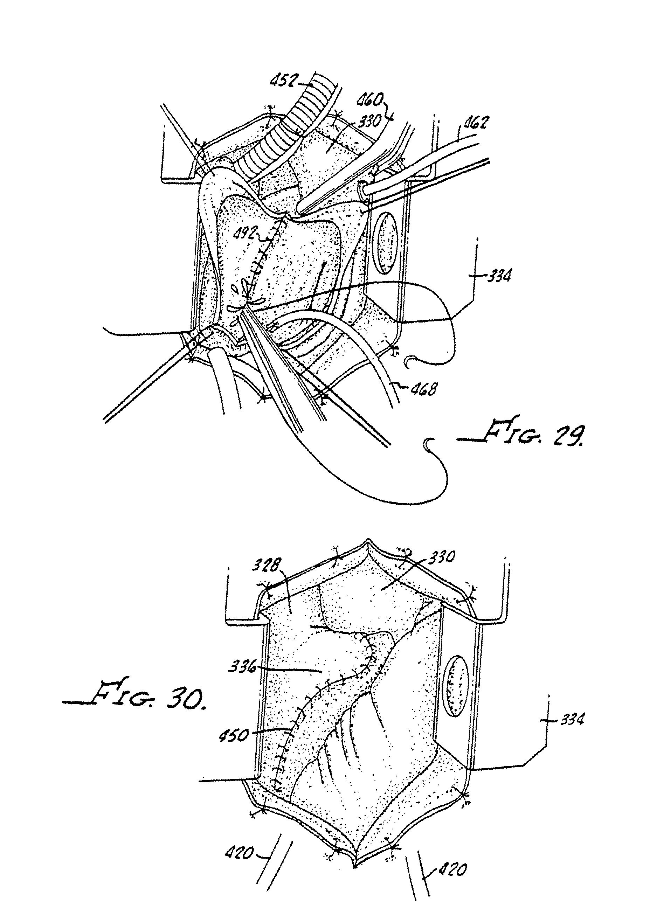

Referring to Figure 34, sutures 526 are placed at the top of each commissure 528 of the valve 524 and draped under tension outside the wound so as to elevate the valve 524, retract the aorta 330 and give a normal anatomical orientation to the aortic root. Cardioplegia is then introduced into one of the coronary ostia 522 with an antegrade cardioplegia catheter 530. The cardiac activity of the heart then progressively diminishes until the heart ceases beating altogether.

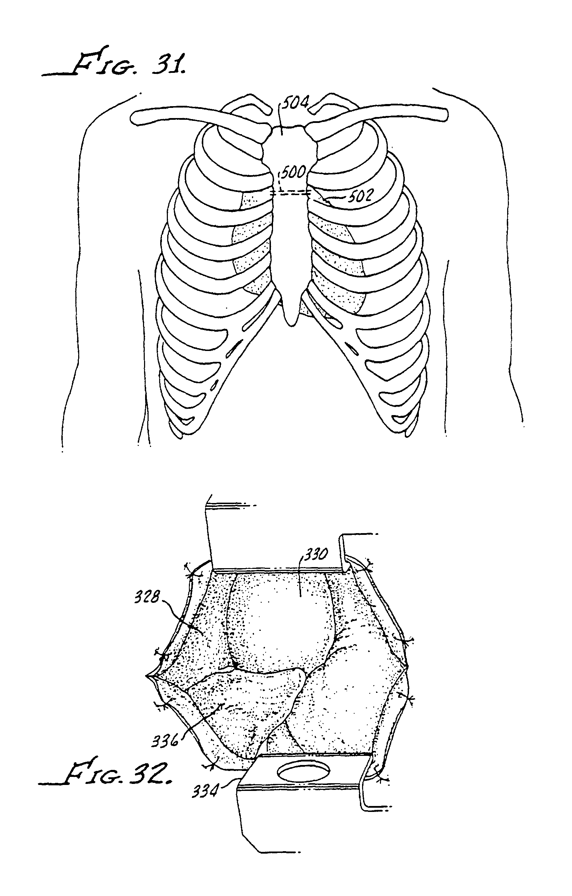

Referring to Figure 35, replacement of the aortic valve is effected by excising the native aortic valve tissue and placing sutures 532 around the annulus of the aortic root. The sutures 532 are then placed through the sewing ring of the aortic valve prosthesis 534 which is attached to a valve holder 536. The prosthesis 534 is then guided into location, the sutures 532 tightened and tied and the holder 536 removed.

Referring to Figures 35 and 36, the sutures 526 through the commissures 528 are maintained in tension until closure of the aorta 330 is begun in order to enable proper exposure of the field. Closure of the aorta 330 is begun by applying a single layer of 4-0 Prolene or other suitable material to bring the edges of the incision together. The sutures 526 attached to the commissures 528 are then cut.

Prior to completion of the closure of the aorta 330, care is taken to remove air from the left ventricle. The lungs are inflated and blood is allowed to flow into the aorta 330 by releasing the clamp 516 which enables air to escape through the remaining open portion of the incision, which portion is held open with a tool 540. The completeness of the air removal is monitored by echocardiography.

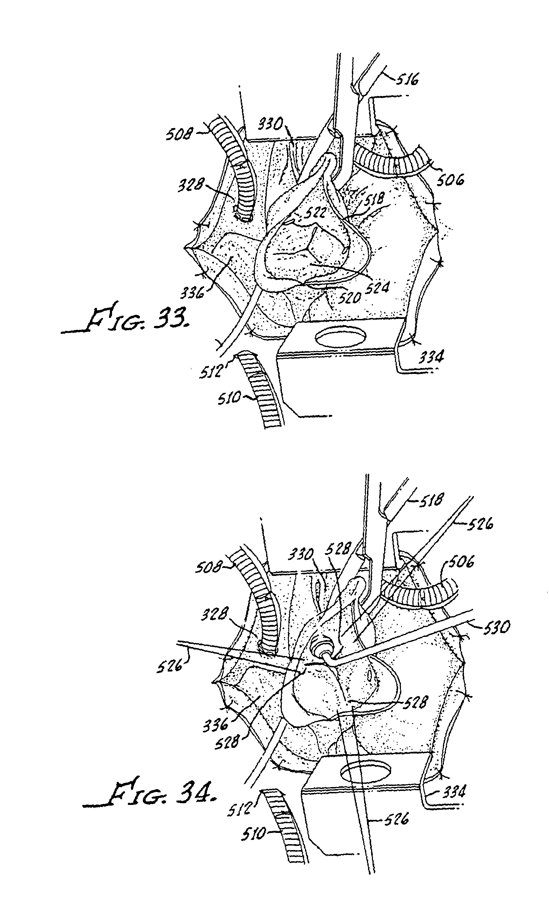

Referring to Figure 37, the patient is further weaned from bypass and closure of the incision 518 is completed. Assuming normal cardiac function returns, the patient is then decannulated and the wounds from the cannulation repaired and closed. Two atrial and two ventricular pacing wires 542, 544 are placed percutaneously into the chest for pacing the heart if necessary. A pleural chest tube 422 is also placed in the chest.

The retractor 334 is then removed and the sternum 504 is closed with monofilament wire or any other suitable material. The incision 500 is then closed by reapproximating the muscles, the subcutaneous tissue and skin, in layers.

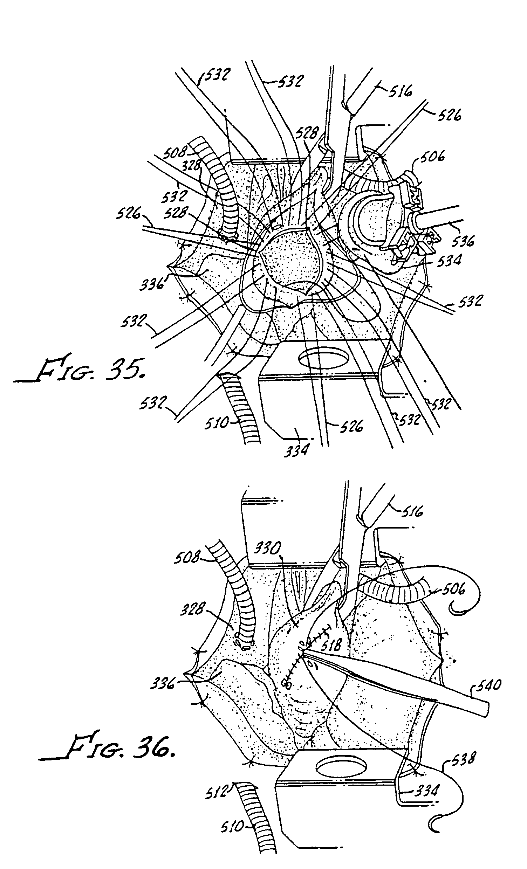

Referring to Figure 38, yet another approach to minimally invasive surgery in accordance with the present invention is disclosed. In this approach, the patient is prepared for surgery in the same manner as described previously, i.e., the patient is anesthetized and intubated and defibrillator patches and a transesophageal cardiography probe are placed on or in the patient, however, with this approach the incision is an 8-10 centimeter incision 602 that is made beginning half-way between the sternal notch 604 and a region slightly above (i.e., 0.5 to 2.0 cm) the sternal notch (also known as the angle of Louie 606). The incision is carried down to the sternum 605 using cautery. Then the sternum 605 is opened from the sternal notch 604 to the third or fourth interspace 608, 610 and extended into that space on the right.

Referring to Figure 39, the wound edges are then sutured back to the wound drape to expose the surgical field. A cannula 612 is then sewn to the wound edge which floods the surgical field with carbon dioxide. Since carbon dioxide is heavier than air, introduction of the carbon dioxide tends to displace oxygen and nitrogen from the surgical field. Moreover, any bubbles of carbon dioxide are rapidly absorbed. In a preferred embodiment, the carbon dioxide is introduced at a rate of 6 liters per minute.

Another aspect of the incision and procedure of the present invention, which can also be used with the other types of incisions previously disclosed, is the use of vacuum-assisted venous drainage. Several types and embodiments of such systems have been previously disclosed herein. In a preferred embodiment, 5333 to 6666 Pa (forty to fifty millimeters of mercury) negative pressure is placed on the venous reservoir. Vacuum assisted drainage also enables the use of reduced size venous cannulae which improves the space constraints in the surgical field. It also has the advantage of providing a drier surgical field and reducing the surgical priming volume of the cardiopulmonary bypass machine by eliminating the need to prime the venous lines.

As with the other incisions and procedures previously discussed, this incision and procedure can be used for both aortic and mitral valve surgery (as well as other surgery on other heart structures). We will discuss first the procedure for conducting aortic valve surgery.

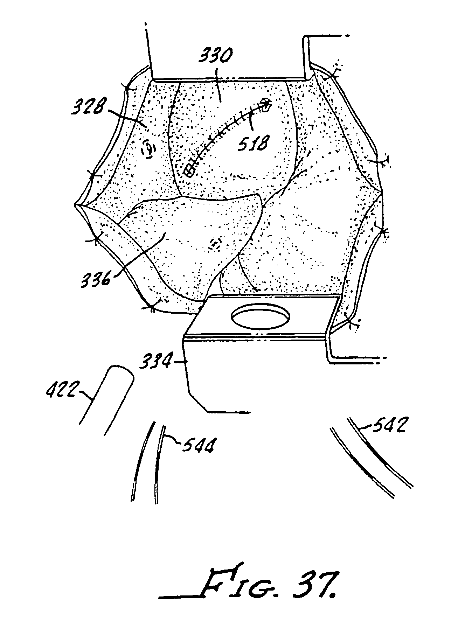

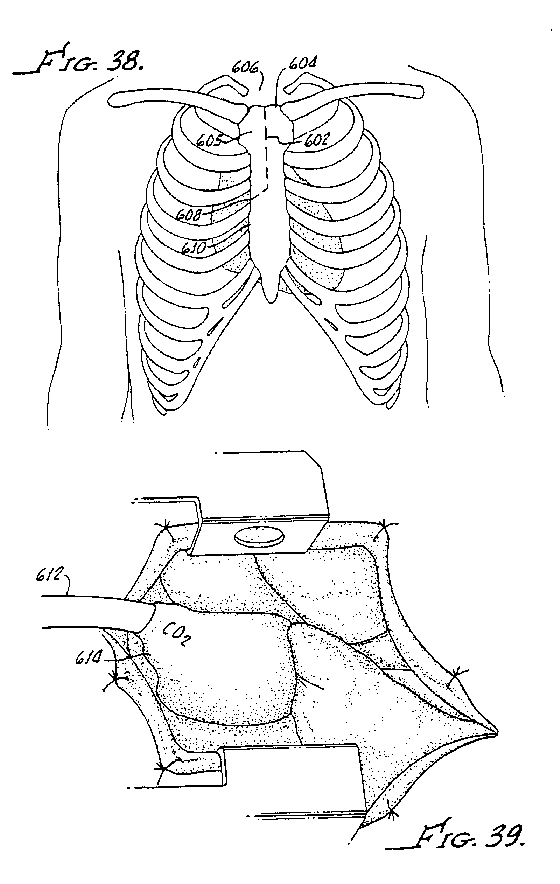

Referring to Figure 40, the aorta 628 is cannulated for arterial return at the pericardial reflection with an arterial return cannula 616 and venous drainage obtained by at least one venous cannula 618 placed in the right atrial appendage 624. A retrograde cardioplegia cannula 622 is placed in the right atrium 626 and directed into the coronary sinus (not shown). The aorta 628 is cross-clamped with an aortic clamp 620 and an oblique incision is made in the aorta 628 which is extended into the noncoronary sinus 625, thus exposing the aortic valve 623.
Referring to Figure 41, sutures 630 are placed at the top of each commissure 632 of the aortic valve 623 and suspended from the drapes under tension. This serves to elevate the valve 623, retract the aorta 628, and gives normal physiologic orientation to the aortic root. Cardioplegia is then injected directly into the coronary ostia 636 with a cardioplegia delivery device 634.

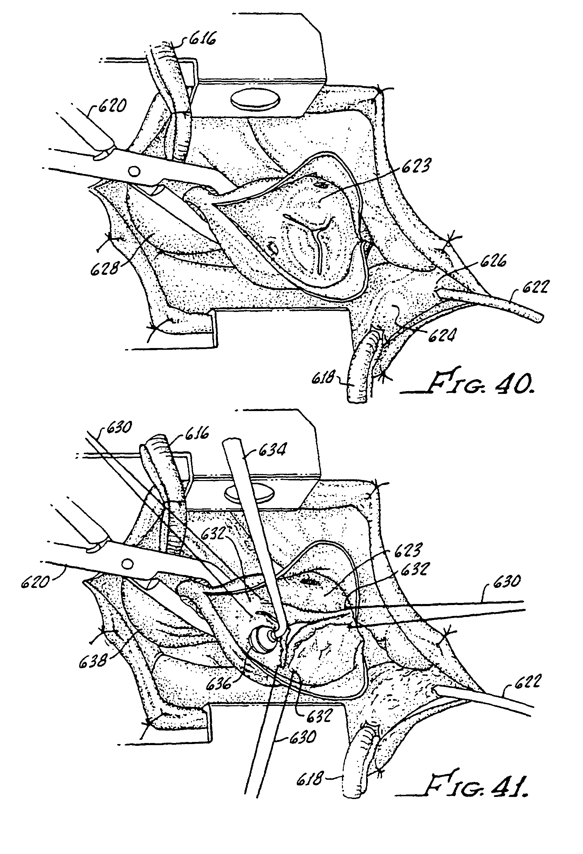

Referring to Figures 42 and 43 the aortic valve 623 is excised and sutures 638 are placed through the annulus and subsequently through the aortic prosthesis 640 and tied. Tension is maintained on the sutures in the valve prosthesis 640 until closure of the aorta 628 has been started. This tension improves the exposure of the most difficult to reach portion of the incision in the non-coronary sinus 625. Sutures 638 in the valve prosthesis 640 are then cut and the aorta 628 closed with single layer of 4-0 Prolene.

Prior to closure, the lungs are inflated which drives air out of the left ventricle and aorta 628. Echocardiography is used to ensure the removal of air and a small cupula 644 is created in the ascending aorta 628 to trap air as it exits the left ventricle. De-airing has been greatly facilitated by flooding the field with carbon dioxide as previously described.

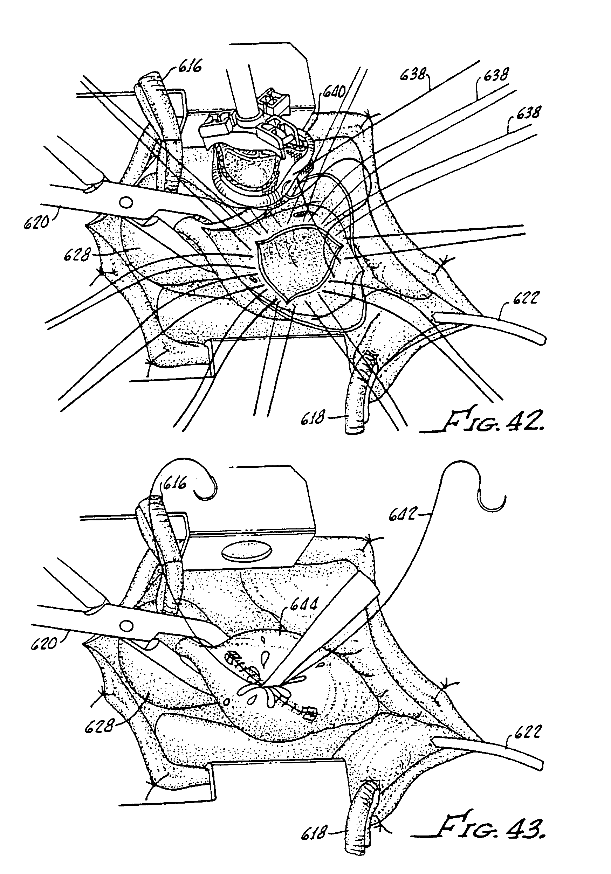

Referring to Figure 44, at completion of the surgery, the patient is decannulated and two atrial and two ventricular wires 646, 648 are placed for use in monitoring and addressing fibrillation and pacing. The sternum is then closed with monofilament wire (Fig. 45) and the wound is closed in layers. Lastly, referring to Figure 45, a right angle chest tube 650 is placed in the patient that lies on top of the diaphragm and a straight tube 652 is inserted directly into the pericardial sac. These tubes are necessary in order to ensure adequate drainage of the mediastinum and right pleura.

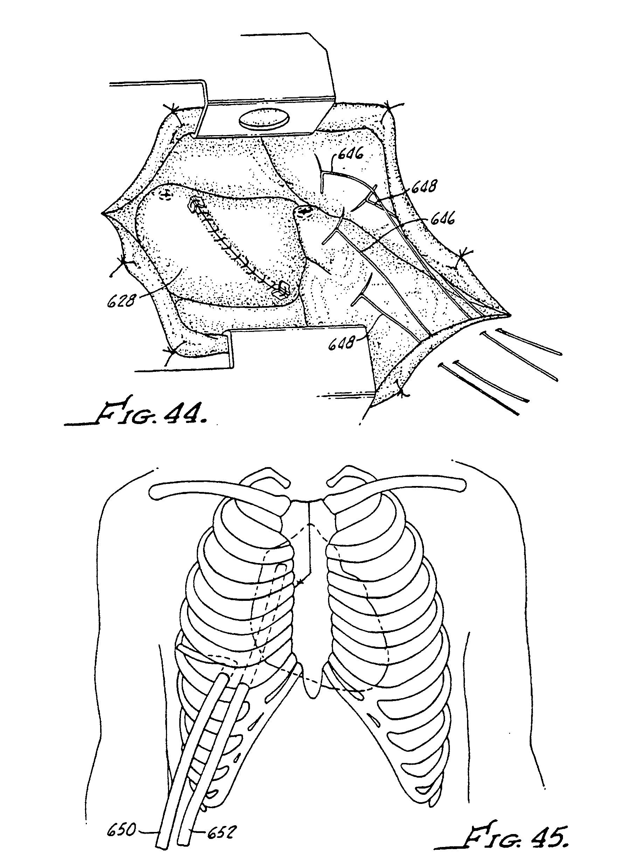

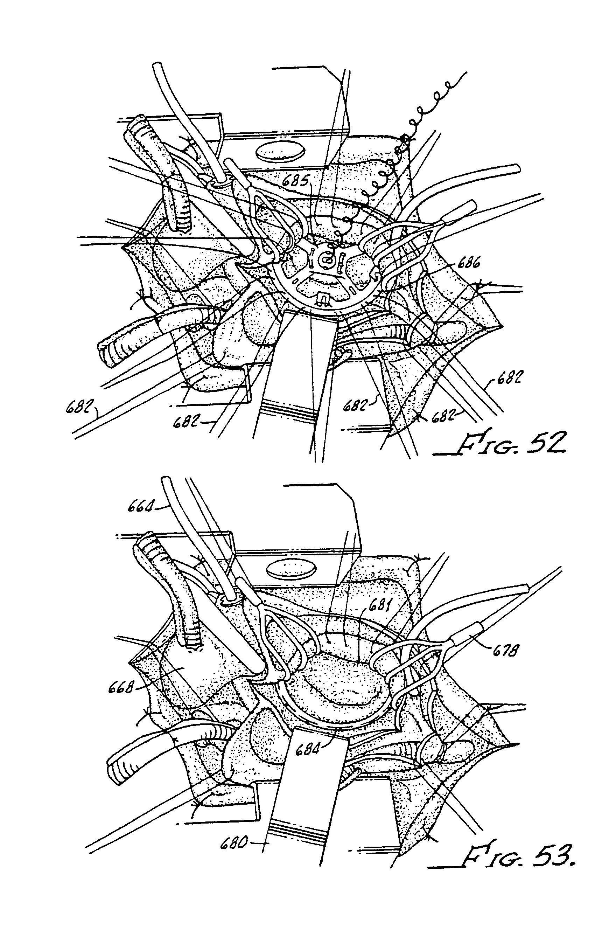

Turning next to repair of the mitral valve, reference is made to Figures 46-55 wherein after the same incision is made as disclosed in Figure 38, the superior vena cava 658 is cannulated with a 20 French cannula 656 and the inferior vena cava 660 cannulated with a similar venous cannula 662. The ascending aorta 628 is cannulated at the pericardial reflection. The small sizes of these cannulae prevent the cannulae from being an obstacle during the surgical procedure.

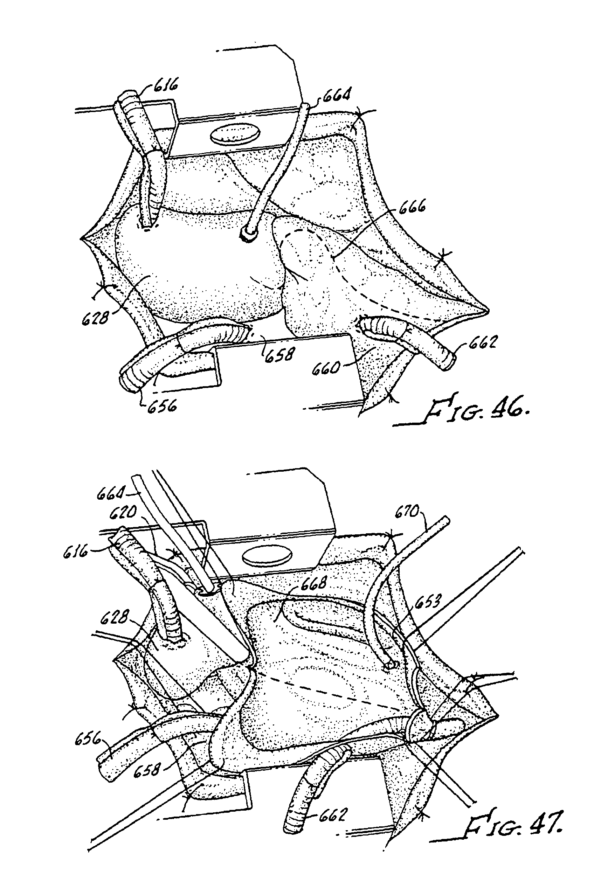

Referring to Figure 47, after the aorta has been clamped with the aortic clamp 620 and cardiac arrest achieved with cardioplegia through the cardioplegia cannula 664, the right atrium is opened, the fossil ovales 671 is incised and the left heart is then decompressed. A purse string is placed around the coronary sinus 673 and a retrograde cardioplegia catheter 670 is then placed in the coronary sinus 673. The incision in the right atrium 624 is extended through the fossa ovales 671 and onto the dome 675 of the left atrium between the superior vena cava 658 and the aorta 628.

Referring to Figure 48, a plurality of pledgetted mattress sutures 672 are placed in the interatrial septum 674 and placed in traction. This retracts the intratrial septum 674 and enhances visualization of the mitral valve 676.

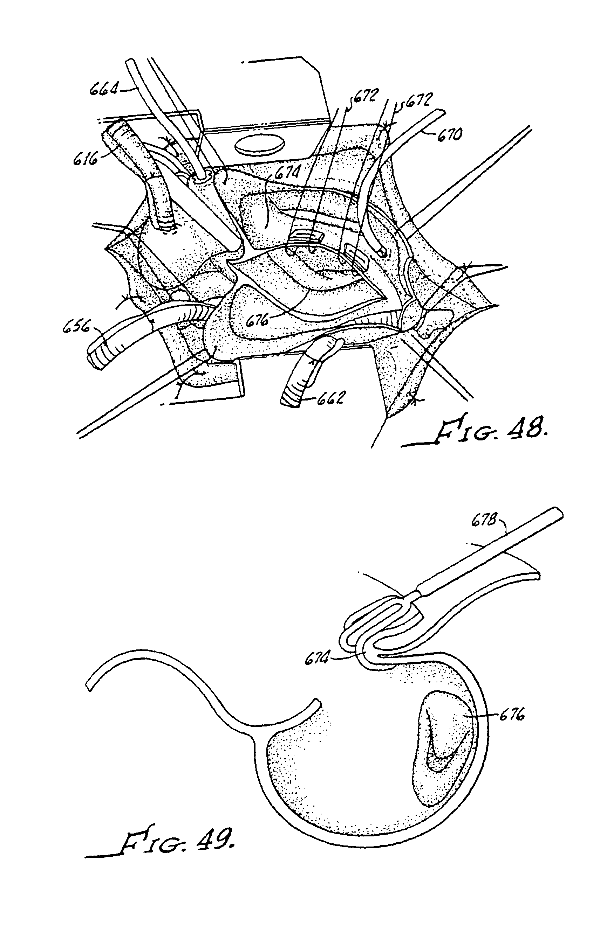

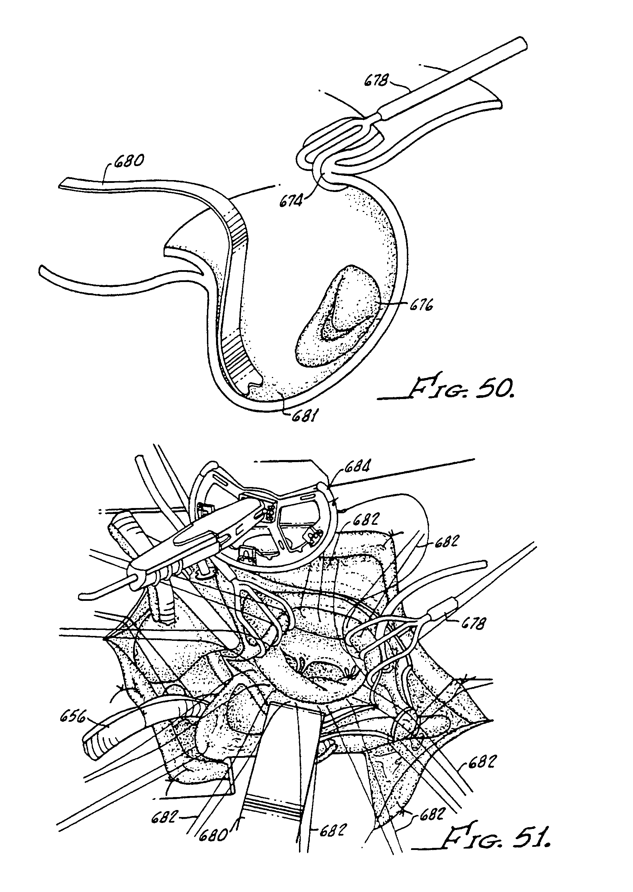

Referring to Figure 49, it is desired to maximize the exposure of the mitral valve 676 in order to ease the surgical technique. Hand-held retractors 678 can be placed on the septum 674 to improve exposure, however, such retractors 678 cannot be pulled anteriorly due to the presence of the sternum (which remains intact). As a result, as shown in Figures 50 and 51, a retractor known as a Harrington retractor 680 is placed in the left atrium 681 and traction placed laterally towards the surgeon. The use of the Harrington retractor 680 in this manner helps position the mitral valve 676 into the direct view of the surgeon.

Referring next to Figures 51, 52 and 53 after repair of the valve mechanism, an annuloplasty is performed. Horizontal mattress sutures 682 of multifilament 2-0 are placed beginning at the fibrous trigone 683 and proceeding around the posterior annulus of the opposite fibrous trigone 683. Sutures 682 are then passed through the annuloplasty band 684 at regular intervals. The handle is then released and the annuloplasty band 68 slid into position. The sutures are tied and the stent 685 is removed leaving the annuloplasty band 684 in place.

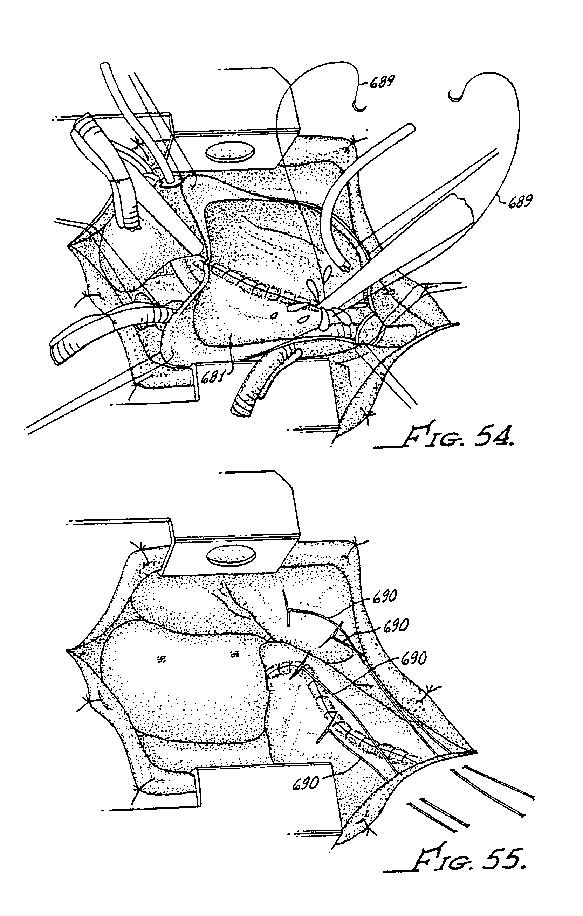

Referring to Figure 54, the incision in the left atrium 681 is closed using continuous 4-0 Prolene 689. Prior to closure of the incision if the interatrial septum 674, air is removed from the left atrium 681 by inflating the lungs. The sutures are then tied. De-airing of the left ventricle is facilitated by gentle suction of the cardioplegia cannula 664 in the ascending aorta 628 prior to and after removal of the aortic clamp 620.

Referring to Figure 55, the incision in the right atrium 624 is then closed and cardiac function of the heart is restored. Cannulae are removed and then pacemaker wires 690 and chest tubes (See Figure 45) are placed in the chest as previously described. The wound is closed in layers with monofilament stainless steel sutures to the sternum and continuous layers of absorbable sutures to the subcutaneous tissue and skin.

The minimally invasive valve surgery in accordance with the present invention simplifies the cardiac surgery for surgeons and provides beneficial results for patients. The operative procedure allows for a relatively small, e.g., approximately 8-10 centimeter, incision that makes opening and closing of the chest easier and faster without compromising the surgical exposure or access to the surgical area. Performing repairs or replacements through an incision in accordance with the present invention simplifies the surgical technique without increasing the difficulty of the procedure or the technical ability required to perform aortic valve surgery. Further, the smaller incision employed in the procedure results in less bleeding, and a lesser area to become infected.
Moreover, not only does the smaller incision tend to cause less incisional pain in patients, the absence of traumatic retraction and the strain placed on the ribs from a gross thoracotomy tends to also account for lower incisional pain. Without incisional pain, patients require less postoperative analgesia and are more easily ambulated allowing for earlier discharge from the hospital. Decreased patient morbidity as a result of decreased postoperative discomfort tends to result in shorter length of hospital stays.

## Claims

1. A cardiopulmonary by-pass system comprising:
a first cannula for providing arterial return blood supply to the patient;
a second cannula (34) for providing venous drainage from the patient;
a venous reservoir (12) closed to atmosphere having a blood inlet (42) connected to said second cannula; **characterised in that** there is
a vacuum supply (18) connected to said venous reservoir for providing a predetermined desired vacuum pressure range of -9332 Pa to -3333 Pa (-70 to -25 mm Hg) within said venous reservoir;
said second cannula having a diameter of 28 Fr or less so as to minimize the space consumed by said second cannula when placed within a surgical field, a reduced cross-sectional area sized to maintain sufficient drainage from said patient under said vacuum pressure range within said venous reservoir.

2. The system of Claim 1, wherein said venous reservoir (12) is hard-shelled (202) and has a blood outlet (44) for removing blood from the reservoir and a vacuum inlet (40) for supplying a vacuum to the reservoir.

3. The system of Claim 1, additionally including a vacuum regulator subassembly (14) for manually setting said predetermined desired vacuum pressure range.

4. The system of Claim 1, additionally including a valve subassembly (16) for manually enabling and disabling a vacuum from being supplied to said reservoir.

5. The system of Claim 4, wherein said valve subassembly (16) additionally includes a check valve (88) for automatically disabling a vacuum from being supplying to said reservoir (12).

6. The system of Claim 1, wherein said venous reservoir (12) additionally includes a cardiotomy inlet for supplying vented and
suctioned blood removed under a vacuum during cardiopulmonary by-pass to said reservoir.

7. The system of Claim 1, wherein said vacuum supply (18) is provided from a wall vacuum source (20).

8. The system of Claim 1, wherein said second cannula (34) has a diameter of about 22 Fr or less.

9. The system of Claim 1, wherein said first cannula has a diameter of about 28F or less.

10. The system of Claim 9, wherein said first cannula has a diameter of about 22F or less.

11. The system of Claim 1, wherein said venous reservoir (12) has a blood outler (44), the system including a patient support, unit for receiving blood from said reservoir blood outlet, treating and returning revitalized removed blood under positive pressure, wherein said patient support unit for creating and returning revitalized removed blood includes three positive pressure pumps (26,26a,26b,26c,26d), said first pump for removing blood from said reservoir, said second pump for removing blood from a patient via vent and suction lines, and said third pump for supplying cardioplegia fluid to a patient.

12. The system of Claim 1, including a conduit (36a) extending between the source of vacuum and the reservoir (12), and a vacuum stabilizer positioned in the conduit, the vacuum stabilizer allowing air into the conduit from the exterior thereof to modulate extreme changes in pressure within the conduit, but preventing air from escaping from the conduit.

13. The system of Claim 1, wherein the reservoir (12) has a flexible blood container (212), the system further including a rigid housing (202) surrounding the flexible container, the system including a conduit (36a) attached to create a vacuum within the housing.

14. The system of Claim 13, further including a pressure relief valve in fluid communication with the conduit (36a) and limiting the magnitude of vacuum in the housing.

15. The system of Claim 1, including a conduit (36a) extending between the source of vacuum and the reservoir (12), and a moisture trap (86, 90) in fluid communication with the conduit and serving to collect fluids drawn from the reservoir before reaching the vacuum source.

16. The system of Claim 14, further including a pressure relief valve in fluid communication with the conduit (36a) and limiting the magnitude of vacuum in the reservoir.

17. The system of Claim 1, wherein said venous reservoir (12) is a hard-shelled container (202) shaped to contain the blood entering through the blood inlet which forms a blood surface within an interior space of the reservoir, the system further including;
a blood outlet (44) in the container;
a vacuum port (69) in the reservoir adapted to be connected to the vacuum source; and
a flexible air impermeable membrane (212) mounted within the container and defining a closed space sealed from the interior space of the container, the membrane having sufficient flexibility so that the closed space expands into the interior space upon a vacuum being drawn within the container, the membrane configured to expand and contact the blood surface.

18. The system of Claim 17, wherein the reservoir (12) comprises a generally cylindrical container (202) having a cap (214) and a central venous chamber into which venous blood flows from the blood inlet, the membrane being positioned in a space surrounding the venous chamber and within the container.

19. The system (12) of Claim 18, wherein an exterior surface of the venous chamber is defined by a generally cylindrical defoamer.

20. The system of Claim 1, further comprising:
a rigid, sealed outer housing (202);
the reservoir (12) comprising a flexible, blood impermeable container (212) within the housing;
a conduit 36a attached to the blood inlet port and in communication with the interior of the reservoir, the conduit passing through a sealed opening in the housing and being connected to a source of venous blood;
a second conduit (36b) extending between the vacuum source and the interior of the housing through a sealed opening; and
a pressure regulator (62) between the vacuum conduit and vacuum source.

21. The system of Claim 20, further including an air permeable membrane forming a portion of the flexible container for venting air from within the container to the housing interior.

22. The system of Claim 20, further including a third conduit (36c) extending between a source of vacuum and in communication with the interior of the container through a sealed opening in the housing; and
a pressure regulator (62) between the third conduit and vacuum source.

## Patentansprüche

1. Kardiopulmonares By-Pass-Systern, das folgendes aufweist:
- eine erste Kanüle, um eine arterielle Rückführungs-Blutversorgung zu dem Patienten zu bilden;
- eine zweite Kanüle (34), um eine Venendrainage von dem Patienten zu bilden;
- ein Venenreservoir (12), das gegenüber der Atmosphäre abgeschlossen ist und einen Bluteinlaß (42) besitzt, der an die zweite Kanüle angeschlossen ist,
**dadurch gekennzeichnet,**
**daß** eine Vakuumzuführung (18) an das Venenreservoir angeschlossen ist, um einen vorgegebenen gewünschten Unterdruck von -9332 Pa bis -3333 Pa (-70 Torr bis -75 Torr) innerhalb des Venenreservoirs auszubilden;
wobei die zweite Kanüle einen Durchmesser von 28 Fr oder weniger besitzt, um den Raum minimal zu machen, der von der zweiten Kanüle eingenommen wird, wenn sie sich in einem Opetationsfeld befindet, und eine reduzierte Querschnittsfläche aufweist, die so bemessen ist, daß eine ausreichende Drainage von dem Patienten bei dem Unterdruckbereich innerhalb des Venenreservoirs aufrechterhalten wird.

2. System nach Anspruch 1,
wobei das Venenreservoir (12) eine harte Schale (202) besitzt und einen Blutauslaß (44) zum Abführen von Blut aus dem Reservoir und einen Vakuumeinlaß (40) zum Anlegen eines Vakuums an das Reservoir aufweist.

3. System nach Anspruch 1,
das ferner eine Vakuumregulier-Unteranordnung (14) aufweist, um den vorgegebenen gewünschten Unterdruckbereich manuell einzustellen.

4. System nach Anspruch 1,
das ferner eine Ventil-Unteranordnung (16) aufweist, um ein Vakuum, das an das Reservoir angelegt wird, manuell einzuschalten und abzuschalten.

5. System nach Anspruch 4,
wobei die Ventil-Unteranordnung (16) zusätzlich ein Rückschlagventil (88) aufweist, um automatisch ein Vakuum abzuschalten, das an das Reservoir (12) angelegt wird.

6. System nach Anspruch 1,
wobei das Venenreservoir (12) zusätzlich einen Kardiotomie-Einlaß aufweist, um belüftetes und abgesaugtes Blut, das unter einem Vakuum während eines kardiopulmonaren By-Passes entfernt worden ist, dem Reservoir zuzuführen.

7. System nach Anspruch 1,
wobei die Vakuumzuführung (18) von einer Wand-Vakuumquelle (20) aus vorgesehen ist.

8. System nach Anspruch 1,
wobei die zweite Kanüle (34) einen Durchmesser von etwa 22 Fr oder weniger besitzt.

9. System nach Anspruch 1,
wobei die erste Kanüle einen Durchmesser von etwa 28 Fr oder weniger besitzt.

10. System nach Anspruch 9,
wobei die erste Kanüle einen Durchmesser von etwa 22 Fr oder weniger besitzt.

11. System nach Anspruch 1,
wobei das Venenreservoir (12) einen Blutauslaß (44) besitzt, wobei das System eine Patienten-Unterstützungseinheit aufweißt, um Blut von dem Reservoir-Blutauslaß aufzunehmen, und entferntes Blut unter Überdruck zu behandeln und das Blut in revitalisierter Form zurückzuführen,
wobei die Patienten-Unterstützungseinheit zum Behandeln und Zurückführen von revitalisiertem, entfernten Blut drei Überdruckpumpen (26, 26a, 26b, 26c, 26d) aufweist, wobei die erste Pumpe zum Entfernen von Blut aus dem Reservoir, die zweite Pumpe zum Entfernen von Blut von dem Patienten über Belüftungs- und Saugleitungen, und die dritte Pumpe zum Zuführen von Cardioplegie-Fluid zu einem Patienten dient.

12. System nach Anspruch 1,
das eine Leitung (36a), die sich zwischen der Vakuumquelle und dem Reservoir (12) erstreckt, und einen Vakuumstabilisator aufweist, der in der Leitung angeordnet ist, wobei der Vakuumstabilisator die Einführung von Luft in die Leitung von deren Außenseite ermöglicht, um extreme Druckänderungen innerhalb der Leitung zu modulieren, aber den Austritt von Luft aus der Leitung verhindert.

13. System nach Anspruch 1,
wobei das Reservoir (12) einen flexiblen Blutbehälter (212) aufweist,
wobei das System ferner ein starres Gehäuse (202) besitzt, welches den flexiblen Behälter umgibt, und
wobei das System eine Leitung (36a) aufweist, die in der Weise angeschlossen ist, daß ein Vakuum bzw. Unterdruck innerhalb des Gehäuses erzeugt wird.

14. System nach Anspruch 13,
das ferner ein Druckablaßvertil aufweist, das mit der Leitung (36a) in Fluidverbindung steht und den Wert des Vakuums bzw. des Unterdrucks in dem Gehäuse begrenzt.

15. System nach Anspruch 1,
das eine Leitung (36a), die sich zwischen der Vakuumquelle und dem Reservoir (12) erstreckt, und eine Feuchtigkeitsfalle (86, 90) aufweist, die mit der Leitung in Fluidverbindung steht und die dazu dient, aus dem Reservoir abgezogene Fluide zu sammeln, bevor sie die Vakuumquelle erreicht.

16. System nach Anspruch 14,
das ferner ein Druckablaßventil aufweist, das mit der Leitung (36a) in Fluidverbindung steht und den Wert des Vakuums bzw. des Unterdrucks in dem Reservoir begrenzt.

17. System nach Anspruch 1,
wobei das Venenreservoir (12) ein Container (202) mit harter Schale ist, der so geformt ist, daß er das Blut aufnimmt, welches durch den Bluteinlaß eintritt, welches eine Blutoberfläche innerhalb eines Innenraumes des Reservoirs bildet,
wobei das System ferner folgendes aufweist:
- einen Blutauslaß (44) in dem Behälter;
- einen Vakuumanschluß (69) in dem Reservoir, der dazu ausgelegt ist, an die Vakuumquelle angeschlossen zu werden; und
- eine luftundurchlässige flexible Membran (212), die innerhalb des Containers angebracht ist und die einen geschlossenen Raum bildet, der gegenüber dem Innenraum des Containers abgedichtet ist, wobei die Membran eine ausreichende Flexibilität besitzt, so daß sich der geschlossene Raum in den Innenraum ausdehnt, wenn ein Unterdruck innerhalb des Containers erzeugt wird, wobei die Membran so ausgelegt ist, daß sie sich ausdehnt und mit der Blutoberfläche in Kontakt steht.

18. System nach Anspruch 17,
wobei das Reservoir (12) einen im allgemeinen zylindrischen Container (202) aufweist, der eine Abdeckung (214) und eine zentrale Venenkammer besitzt, in welche venöses Blut aus dem Bluteinlaß strömt, wobei die Membran in einem Raum, der die Venenkammer umgibt, und innerhalb des Containers positioniert ist.

19. System (12) nach Anspruch 18,
wobei eine Außenoberfläche der Venenkammer von einer im allgemeinen zylindrischen Schaumbeseitigungseinrichtung gebildet ist.

20. System nach Anspruch 1,
das ferner folgendes aufweist:
- ein steifes, abgedichtetes äußeres Gehäuses (202);
- wobei das Reservoir (12) einen flexiblen, blutundurchlässigen Container (212) innerhalb des Gehäuses aufweist;
- eine Leitung (36a), die an dem Bluteinlaßanschluß angebracht ist und mit dem Innenraum des Reservoirs in Verbindung steht, wobei die Leitung durch eine abgedichtete Öffnung in dem Gehäuse hindurchgeht und an eine Quelle für venöses Blut angeschlossen ist;
- eine zweite Leitung (36b), die sich zwischen der Vakuumquelle und dem Innenraum des Gehäuses durch eine abgedichtete Öffnung erstreckt; und
- eine Druckreguliereinrichtung (62) zwischen der Vakuumleitung und der Vakuumquelle.

21. System nach Anspruch 20,
das ferner eine luftdurchlässige Membran aufweißt, die einen Bereich des flexiblen Containers bildet, um Luft aus dem Raum innerhalb des Containers in das Innere des Gehäuses abzulassen.

22. System nach Anspruch 20,
das ferner folgendes aufweist:
- eine dritte Leitung (36c), die sich zwischen einer Vakuumquelle und in Verbindung mit dem Innenraum des Containers durch eine abgedichtete Öffnung in dem Gehäuse erstreckt; und
- eine Druckreguliereinrichtung (62) zwischen der dritten Leitung und der Vakuumquelle.

## Revendications

1. Système de circulation extracorporelle comprenant :
une première canule pour fournir une circulation artérielle de retour au patient ;
une deuxième canule (34) pour fournir un drainage veineux à partir du patient ;
un réservoir veineux (12) fermé à l'atmosphère ayant une entrée de sang (42) reliée à ladite seconde canule ; **caractérisé en ce qu'**il existe
une alimentation en vide (18) reliée audit réservoir veineux pour fournir une plage de dépression souhaitée prédéterminée allant de -9332 Pa à -3333 Pa (-70 à -25 mm Hg) au sein dudit réservoir veineux ;
ladite seconde canule présentant un diamètre de 28 Fr ou moins afin de réduire au minimum l'espace consommé par ladite seconde canule une fois placée au sein d'un champ chirurgical, une section transversale réduite dimensionnée pour maintenir un drainage suffisant à partir dudit patient sous ladite plage de dépression au sein dudit réservoir veineux.

2. Système selon la revendication 1, dans lequel ledit réservoir veineux (12) est rigide (202) et possède une sortie de sang (44) pour retirer le sang du réservoir et une entrée d'aspiration (40) pour alimenter le réservoir en vide.

3. Système selon la revendication 1, comprenant en outre un assemblage de régulateur de vide (14) pour régler manuellement ladite plage de dépression souhaitée prédéterminée.

4. Système selon la revendication 1, comprenant en outre un assemblage de valves (16) pour permettre et empêcher manuellement l'alimentation en vide dudit réservoir.

5. Système selon la revendication 4, dans lequel ledit assemblage de valves (16) comprend en outre un clapet anti-retour (88) pour empêcher automatiquement l'alimentation en vide dudit réservoir (12).

6. Système selon la revendication 1, dans lequel ledit réservoir veineux (12) comprend en outre une entrée de cardiotomie pour fournir un sang aspiré et oxygéné prélevé sous vide au cours de la circulation extracorporelle audit réservoir.

7. Système selon la revendication 1, dans lequel ladite alimentation en vide (18) est fournie à partir d'une source d'aspiration murale (20).

8. Système selon la revendication 1, dans lequel ladite seconde canule (34) présente un diamètre d'environ 22 Fr ou moins.

9. Système selon la revendication 1, dans lequel ladite première canule présente un diamètre d'environ 28 F ou moins.

10. Système selon la revendication 9, dans lequel ladite première canule présente un diamètre d'environ 22 F ou moins.

11. Système selon la revendication 1, dans lequel ledit réservoir veineux (12) possède une sortie de sang (44), le système comprenant une unité de soutien du patient pour recevoir du sang de ladite sortie de sang du réservoir, pour traiter et renvoyer le sang prélevé revitalisé sous pression positive, dans lequel ladite unité de soutien du patient visant à traiter et renvoyer le sang prélevé revitalisé comprend trois pompes à pression positive (26, 26a, 26b, 26c, 26d), ladite première pompe servant à prélever du sang dudit réservoir, ladite deuxième pompe servant à prélever le sang d'un patient via des conduites d'aspiration et de ventilation et ladite troisième pompe servant à approvisionner le patient en liquide de cardioplégie.

12. Système selon la revendication 1, comprenant un conduit (36a) se prolongeant entre la source d'aspiration et le réservoir (12) et un stabilisateur d'aspiration placé dans le conduit, le stabilisateur d'aspiration laissant passer de l'air dans le conduit depuis l'extérieur de celui-ci afin de moduler des changements extrêmes de pression dans le conduit, mais en empêchant l'air de s'échapper du conduit.

13. Système selon la revendication 1, dans lequel le réservoir (12) possède un réservoir de sang flexible (212), le système comprenant en outre un boîtier rigide (202) entourant le réservoir flexible, le système comprenant un conduit (36a) inséré pour créer un vide à l'intérieur du boîtier.

14. Système selon la revendication 13, comprenant en outre une soupape de surpression en communication liquide avec le conduit (36a) et limitant l'importance du vide dans le boîtier.

15. Système selon la revendication 1, comprenant un conduit (36a) se prolongeant entre la source d'aspiration et le réservoir (12), et un collecteur d'humidité (86, 90) en communication liquide avec le conduit et servant à récolter des liquides retirés du réservoir avant d'atteindre la source d'aspiration.

16. Système selon la revendication 14, comprenant en outre une soupape de surpression en communication liquide avec le conduit (36a) et limitant l'importance du vide dans le réservoir.

17. Système selon la revendication 1, dans lequel ledit réservoir veineux (12) est un réservoir rigide (202) formé pour contenir le sang entrant via l'entrée de sang qui forme une surface de sang au sein d'un espace intérieur du réservoir, le système comprenant en outre :
une sortie de sang (44) dans le réservoir ;
un orifice d'aspiration (69) dans le réservoir adapté pour être relié à la source d'aspiration ; et
une membrane flexible imperméable à l'air (212) montée à l'intérieur du réservoir et définissant un espace clos fermé hermétiquement à partir de l'espace intérieur du réservoir, la membrane ayant une flexibilité suffisante de sorte que l'espace clos augmente dans l'espace intérieur lorsque le vide est attiré dans le réservoir, la membrane étant configurée pour augmenter et entrer en contact avec la surface de sang.

18. Système selon la revendication 17, dans lequel le réservoir (12) comprend un réservoir généralement cylindrique (202) ayant un bouchon (214) et une chambre veineuse centrale dans laquelle le sang veineux s'écoule à partir de l'entrée de sang, la membrane étant placée dans un espace entourant la chambre veineuse et au sein du réservoir.

19. Système (12) selon la revendication 18, dans lequel une surface extérieure de la chambre veineuse est définie par un anti-mousse généralement cylindrique.

20. Système selon la revendication 1, comprenant en outre :
un boîtier externe rigide et hermétiquement fermé (202) ;
le réservoir (12) comprenant un réservoir flexible imperméable au sang (212) à l'intérieur du boîtier ;
un conduit (36a) relié à l'orifice d'entrée de sang et en communication avec l'intérieur du réservoir, le conduit passant à travers une ouverture fermée hermétiquement dans le boîtier et étant relié à une source de sang veineux ;
un second conduit (36b) se prolongeant entre la source d'aspiration et l'intérieur du boîtier à travers une ouverture hermétiquement fermée ; et
un régulateur de pression (62) entre le conduit d'aspiration et la source d'aspiration.

21. Système selon la revendication 20, comprenant en outre une membrane perméable à l'air formant une partie du réservoir flexible pour ventiler l'air à partir de l'intérieur du réservoir jusqu'à l'intérieur de boîtier.

22. Système selon la revendication 20, comprenant en outre un troisième conduit (36c) se prolongeant entre une source d'aspiration et en communication avec l'intérieur du réservoir à travers une ouverture hermétiquement fermée dans le boîtier ; et
un régulateur de pression (62) entre le troisième conduit et la source d'aspiration.
